Europäisches Patentamt

European Patent Office

Office européen des brevets

(11) Veröffentlichungsnummer: **0 297 362**
**A2**

## (12) EUROPÄISCHE PATENTANMELDUNG

(21) Anmeldenummer: 88109617.6

(22) Anmeldetag: 16.06.88

(51) Int. Cl.⁴: **C07K 7/10 , A61K 37/02**

Der (Die) Mikroorganismus (Mikroorganismen) ist (sind) bei Deutsche Sammlung von Mikroorganismen unter der (den) Nummer(n) DSM 4160, DSM 4161 hinterlegt worden.

(30) Priorität: 27.06.87 DE 3721240
24.07.87 DE 3724570

(43) Veröffentlichungstag der Anmeldung:
04.01.89 Patentblatt 89/01

(84) Benannte Vertragsstaaten:
AT BE CH DE ES FR GB GR IT LI NL SE

(71) Anmelder: BAYER AG

D-5090 Leverkusen 1, Bayerwerk(DE)

(72) Erfinder: **Bruns, Wolfgang, Dr.**
**Kaiser-Wilhelm-Allee 37**
**D-5600 Wuppertal 1(DE)**
Erfinder: **Ebbers, Jürgen, Dr.**
**Moltkestrasse 65**
**D-5600 Wuppertal 1(DE)**
Erfinder: **Hörlein, Hans-Dietrich, Dr.**
**Paul-Ehrlich-Strasse 20**
**D-5600 Wuppertal 1(DE)**
Erfinder: **Schnabel, Eugen, Dr.**
**Schimmelweg 6**
**D-5600 Wuppertal 11(DE)**
Erfinder: **Schröder, Werner, Dr.**
**Claudiusweg 9**
**D-5600 Wuppertal 1(DE)**

(54) **Human-Aprotinin, dessen Lys-Rest in Position 15 gegen einen anderen protogenen Aminosäurerest ausgetauscht ist.**

(57) Gegenstand dieser Erfindung sind Homologe von Aprotinin deren Aminosäuresequenzen von der des humanen Proteins abgeleitet sind sowie Verfahren zu ihrer Herstellung mit gentechnologischen Methoden und ihre Verwendung als Arzneimittel.

EP 0 297 362 A2

## Human-Aprotinin, dessen Lys-Rest in Position 15 gegen einen anderen protogenen Aminosäurerest ausgetauscht ist

Gegenstand dieser Erfindung sind Homologe von Aprotinin, deren Aminosäuresequenzen von der des humanen Proteins abgeleitet sind sowie Verfahren zu ihrer Herstellung mit gentechnologischen Methoden und ihre Verwendung als Arzneimittel.

Aprotinin, der Basische Pankreatische Trypsin Inhibitor (Kunitz) ist ein seit langem bekannter Serinproteinasen-Inhibitor aus Rinderorganen mit einer breiten inhibitorischen Spezifität. Seine Aminosäuresequenz ist bekannt. Aprotinin wird seit vielen Jahren als Arzneimittel in der Therapie von Krankheiten verwendet, die durch das Fehlen oder einen Mangel an den natürlichen Inhibitoren von mit Trasylol hemmbaren Enzymen ausgelöst und aufrecht erhalten werden (vgl. H. Fritz und G. Wunderer, Arzneimittelforschung 33, 479-494 (1983)).

Zahlreiche Krankheiten sind nun aber bedingt durch einen Überschuß an Enzymen, die nicht mit Aprotinin gehemmt werden können. Eine wichtige Rolle spielen in diesem Zusammenhang die lysosomalen Enzyme und unter diesen insbesondere die Leukozytenelastase. Normalerweise wird sie bei der Ausschüttung in den extrazellulären Raum sofort durch Komplexierung mit den körpereigenen Inhibitoren wie $\alpha_1$-Proteaseninhibitor ($\alpha_1$-PI) - J. Travis und G.S. Salvesen, Ann. Rev. Biochem. (1983) 655-709 -, Antileukoprotease (HUSI I) - H. Schiessler u.a. in: Neutral Proteases of Human Polymorphonuclear Leukocytes (1978) 195-207; K. Havemann und A. Janoff Hrgb., Urban + Schwarzenberg, Baltimore - oder $\alpha_2$-Makroglobulin ($\alpha_2$-M) - G. Salvesen, D. Virca und J. Travis, Ann. N.Y. Acad. Sci. 421, 316-326 (1983) - inaktiviert. Hereditärer $\alpha_1$-PI-Mangel oder oxidative Inaktivierung dieses Inhibitors - J. Travis und G.S. Salvesen, Ann. Rev. Biochem. (1983), 655-709 - oder eine massive Enzymausschüttung, insbesondere von Leukozytenelastase, führen zu einem extensiven Abbau von Bindegewebe und humoralen Funktionsproteinen mit ernsthaften klinischen Symptomen wie Lungenemphysem, Schocklunge, Adult Respiratory Distress Syndrom, Gerinnungsstörungen sowie Nieren- und Leber-Versagen - vgl. M. Jochum u.a. (Hrgb.) - in: Neue Wege in der Entzündungsdiagnostik, PMN-Elastase (1985), GIT-Verlag, Darmstadt; W.W. McGuire u.a., J. Clin. Invest. 69, 543 (1982) sowie C.T. Lee u.a., N. Engl. J. Med. 304, 192-196 (1981) -. Auch bei akuten und chronischen Entzündungen, z.B. bei der rheumatoiden Arthritis ist die verhängnisvolle Rolle der Leukozytenelastase gesichert - K. Klesiek u.a. in: Neue Wege in der Entzündungsdiagnostik, PMN-Elastase (1985) 71-82, GIT-Verlag, Darmstadt -.

In experimentellen Sepsis- und Emphysem-Modellen wurde die therapeutische Wirksamkeit von synthetischen Elastaseinhibitoren - J.C. Powers, Ann. Rev. Respir. Dis. 127, 554-558 (1983) - sowie dem gentechnologisch hergestellten Elastaseinhibitor Eglin c - H.P. Schnebli u.a., Europ. J. Respir. Dis. 66 Suppl. 66-70 (1985) - nachgewiesen. Vor allem in der Langzeittherapie bietet jedoch der Einsatz eines stabilen Elastaseinhibitors humaner Provenienz wegen des fehlenden Allergisierungsrisikos und der besseren Verträglichkeit erhebliche Vorteile.

Bekanntlich kann das Hemmspektrum von Aprotinin durch Austausch des "active-site"-Aminosäureesters qualitativ und quantitativ verändert werden - DE-OS 3 339 693 vom 27.07.1983 bzw. 03.11.1983: H. Tschesche, H.R. Wenzel, R. Schmuck und E. Schnabel sowie EP 87 103 942.3 vom 26.03.1986: E.-A. Auerswald, W. Schröder, E. Schnabel, W. Bruns, G. Reinhardt und M. Kotick -.

So wird beim Einbau von Arginin in Position 15 ein Proteaseninhibitor mit vergleichsweise höheren Affinitäten für humanes anionisches und kationisches Trypsin, humanes Plasmakallikrein sowie humanes Organkallikrein erhalten.

Nach Lasson u.a. - A. Lasson und K. Ohlsson, Scand. J. Gastroenterol. 11, 707 (1984) - spielen die Trypsine eine entscheidende Rolle bei der Pankreatitis. Große physiologische und pathophysiologische Bedeutung haben auch die Kallikreine - R. Geiger in: V. Turk und L. Vitale (Hrgb.) Proteinases and Their Inhibitors, Mladinska kujiga-Pergamon Press, Ljubljana, Oxford (1981) 353-376 -. Ihnen kommt eine übergreifende Funktion in den Regelkreisen der Gerinnung, Fibrinolyse, Komplementaktivierung sowie im Renin-Angiotensinsystem und in Verbindung mit Entzündungsreaktionen zu. -H. Verstraete, Drugs 29, 236 (1985) -. Chronisch rezidive Parotitis ist nach H. Maier und D. Adler - Laryng. Rhinol. Otol. 65, 191 (1986) - durch Organkallikrein bedingt. Plasmakallikrein ist beispielsweise verantwortlich für das hereditäre angioneurotische Ödem - M. Schapira u.a., New England, J. Med. 308, 1050 (1983) - sowie die Reperfusions Injury - R.S. Roy und J.M. McCord in: R.A. Greenwald und G. Cohen (Hrgb.), Oxy Radicals and their Scavenger Systems, Vol. II: Cellular und Medical Aspects, Amsterdam, 1983, Elsevier Science Publishing Co., 145-153 - und bewirkt eine Aktivierung der Leukozyten - M. Schapira, C.F. Scott, C.A. Boxer und R.W. Colman, in: H. Fritz, N. Bach, G. Dietze und G.L. Haberland (Hrgb.) Kinin III, part B 1983, 747-753, New York, Plenum Press -. In der Therapie dieser Krankheiten ist das Arg-15-Homologe dem Aprotinin aufgrund seiner besseren Hemmung überlegen.

Die beim Austausch des Lys-15-Restes gegen Gly, Ala, Leu, Ile oder Val erhaltenen semisynthetischen Aprotininhomologen sowie die gentechnologisch dargestellten, gegebenenfalls zusätzlich in Position 52 Glu tragenden Varianten, sind teilweise potente Hemmer von humaner Leukozytenelastase und eignen sich zur Therapie der früher aufgeführten durch Leukozytenelastase ausgelösten und aufrecht erhaltenen Erkrankungen.

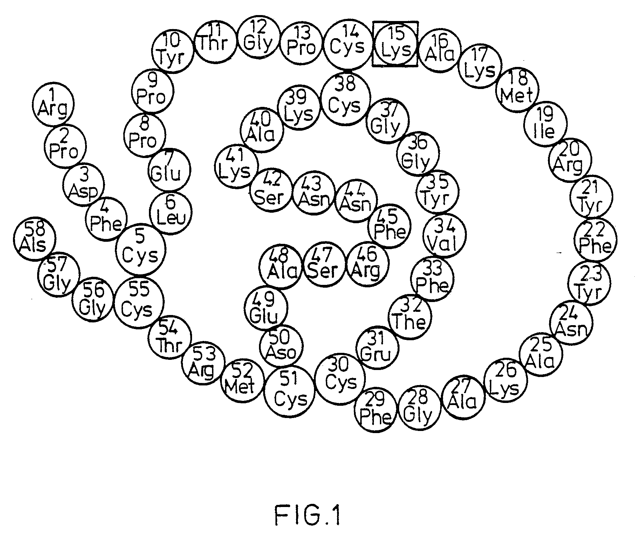

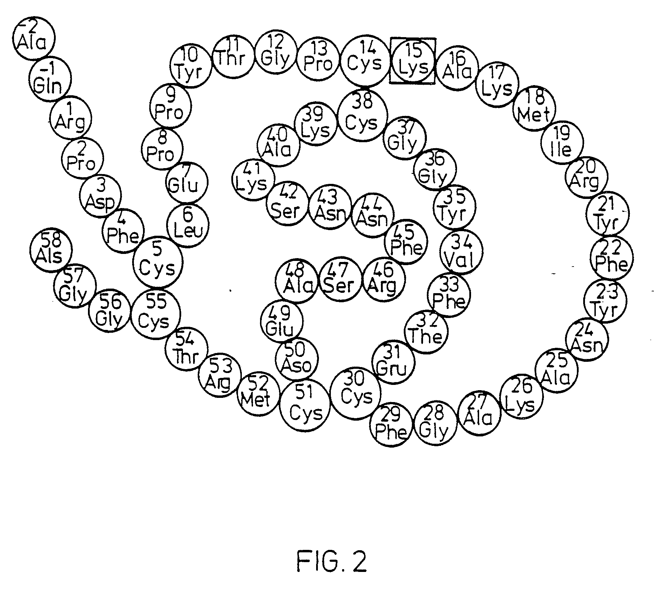

Kürzlich wurden nun aus menschlichem Blutplasma eine Gruppe neuer, dem Aprotinin strukturell eng verwandter polyvalenter Proteaseninhibitoren vom Kunitz-Typ isoliert - E. Fioretti, M. Angeletti, G. Citro, D. Barra und F. Ascoli, J. Biol. Chem. 262, 3586-3589 (1987) -. Die Aminosäuresequenz dieser Inhibitoren, die mit einigen Anti-Aprotinin-Antiseren kreuzreagieren, wurde jetzt aufgeklärt und ist überraschend, wie aus Abb. 1 bzw. 2 ersichtlich, identisch bzw. homolog mit der des bovinen Isoinhibitors II - E. Fioretti, G. Iacopino, M. Angeletti, D. Barra, F. Bossa und F. Ascoli, J. Biol. Chem. 260, 11451-11455 (1985) -.

Unter Human-Aprotinin wird in dieser Anmeldung ein Proteinase Inhibitor vom Kunitz-Typ verstanden, der z.B. aus menschlichem Serum oder tierischen Geweben gewonnen werden kann und eine der in Abb. 1 oder Abb. 2 dargestellten Sequenzen besitzt.

Wie die Abbildungen zeigen, unterscheiden sich die beiden Inhibitoren nur durch eine N-terminale Extension der Peptidkette um 2 Aminosäuren.

Es wurde nun gefunden, daß das Hemmspektrum dieses humanen Inhibitors, der im folgenden als Human-Aprotinin bezeichnet wird, wie das von Aprotinin durch den Austausch des Lys-Restes in Position 15 gegen andere protogene Aminosäurereste modifiziert wird. Die so erhaltene in Position 15 variierten Homologen des humanen Aprotinin sind aufgrund ihrer humanen Provenienz verträglicher als die vom Aprotinin abgeleiteten Homologen. Bei der Anwendung von Aprotinin sind allergische oder pseudoallergische Reaktionen möglich - vgl. Beipackzettel -. Beim Einsatz der erfindungsgemäßen Inhibitoren ist das Risiko allergischer Komplikationen wegen der humanen Provenienz des als Stammsubstanz verwendeten Aprotinin beträchtlich reduziert bzw. nicht gegeben.

In den erfindungsgemäßen, gentechnologisch hergestellten Homologen des humanen Aprotinin können neben dem active-site-Rest in Position 15 ein oder mehrere zusätzliche Aminosäurereste gegen andere protogene Aminosäureste ausgetauscht sein. Bevorzugte Positionen für die Austausche sind:

Position 15 (active-site-Position):

Val, Ile, Leu, Ala, Met, Asn, Gln, Phe, Tyr, Trp oder Arg

Position 16:

Gly, Ser oder Thr

Position 17:

Leu, Val, Ile, Ser, Thr, Asn, Gln, Phe, Tyr oder Arg

Position 18:

Ala, Val, Leu, Ile, Ser, Thr, Asn, Gln, Asp, Glu oder Phe

Position 19:

Val, Ile, Leu, Ala, Thr, Asn oder Gln

Position 34:

Ile, Leu, Ala, Thr, Met, Asn, Gln, Asp, Glu, Phe oder Arg

Position 39:

Val, Ile, Leu, Met, Asn, Gln, Asp, Glu, Phe, Ser, Thr oder Arg

Position 52:

Val, Ile, Leu, Gln, Glu, Thr, Asp, Phe, Lys oder Arg.

Außerdem können in den Homologen die beiden eine Disulfidbrücke bildenden Halbcystinreste in Position 14 und 38 ersetzt sein durch: Gly, Ala, Val, Leu, Pro, Ser, Thr, Asn, Gln, Glu, Lys, Arg oder Phe, wobei die Aminosäurereste in beiden Positionen gleich oder verschieden sein können.

Die erfindungsgemäßen Inhibitoren können außerdem einen Met-Rest in Position⁻¹ enthalten oder eine der Struktur II entsprechende N-terminale Extension durch das Dipeptid Ala-Gln, der ebenfalls ein Met-Rest vorausgehen kann. Gegebenenfalls können die Inhibitoren ein N-terminales Leaderpeptid mit Signal- und/oder Linker-Sequenzen enthalten, die hohe Expression gewährleisten und/oder die Isolierung und/oder Reinigung erleichtern.

Besonders bevorzugt im Rahmen der Erfindung sind

Val-15-Human-Aprotinin,

Val-15-Leu-17-Ile-18-Thr-52-Human-Aprotinin,

Leu-15-Leu-17-Ile-18-Thr-52-Human-Aprotinin,

Val-15-Leu-17-Ile-18-Glu-39-Thr-52-Human-Aprotinin.
und Arg-15-Arg-17-Ile-18-Glu-52-Human-Aprotinin.

Die Hemmspektren einiger erfindungsgemäßer Inhibitoren sind in Tab. 1 zusammengestellt.

Tabelle 1    Hemmspektren einiger erfindungsgemäßer Inhibitoren für verschiedene humane Serinproteinasen ($IC_{50}$-Werte in ng[1])

| Inhibitor | Cathepsin G | Enzym Leukozyten-elastase | Organ-kallikrein | Plasma-kallikrein | Trypsine |
|---|---|---|---|---|---|
| | (7 ng) | (185 ng) | (815 ng) | (270 ng) | (25 ng) |
| Val-15-HA[2] | - | 18 | - | - | - |
| Val-15-Leu-17-Ile-18-HA | + [3] | 18 | - | - | - |
| Val-15-Leu-17-Ile-18-Thr-52-HA | + | 18 | - | - | - |
| Leu-15-Leu-17-Ile-18-Thr-52-HA | 650 | 18 | - | - | - |
| Arg-15-Arg-17-Ile-18-Arg-39-Glu-52-HA | - | - | 67 | 50 | 3,3 |
| Aprotinin | + | + | 67 | 750 | 3,3 |

[1]  bezogen auf Reinprotein

[2]  HA = Human-Aprotinin

[3]  + schwache aber eindeutige Hemmung

Gegenstand der vorliegenden Erfindung sind außerdem die synthetischen Desoxyribonucleinsäuren, die für die erfindungsgemäßen Proteinaseinhibitoren codieren. Proteine mit definierter Aminosäuresequenz können bekanntlich gentechnologisch hergestellt werden. Der Austausch einzelner Aminosäurereste ist in an sich bekannter Weise auf zwei Wegen möglich: 1. ausgehend von der genomischen DNA bzw. cDNA durch eine "site-directed" Mutagenese oder 2. durch Synthese eines Gens, das die entsprechenden Codons enthält.

Methoden zur Expression der heterologen DNA in rekombinanten Mikroorganismen und/oder eukariontischen Zellen sind ebenfalls bekannt.

So wurde bereits gezeigt, daß Aprotinin oder Varianten von Aprotinin in rekombinanten Mikroorganismen unter Verwendung von synthetischen Genen entweder unfusioniert - B. von Wilcken-Bergmann u.a., EMBO J. 5, 3219 (1986) -oder als Fusionsproteine mit dem bakteriellen $\beta$-Galaktosidase-Gen - E.-A. Auerswald u.a., Europäische Patentanmeldung 27 103 942.3 vom 26.03.1986 - exprimiert werden können. Es ist weiterhin bekannt, daß die natürliche Coding-Region von Aprotinin als Fusion mit der Signalsequenz der alkalischen Phosphatase exprimiert werden kann - S. Anderson und I.B. Kingston, Proc. Nat. Acad. Sci., USA 80, 6368 (1983) -. Auch das Gen des bovinen Isoinhibitor II, der dem humanen Aprotinin homolog ist, wurde isoliert, jedoch nicht exprimiert -C.B. Marks u.a., J. Biol. Chem. 261, 7115 (1986) -. Homologe bzw. Varianten dieses Inhibitors mit Austausch des active-site-Aminosäurerests in Position 15 sind bis jetzt noch nicht bekannt geworden.

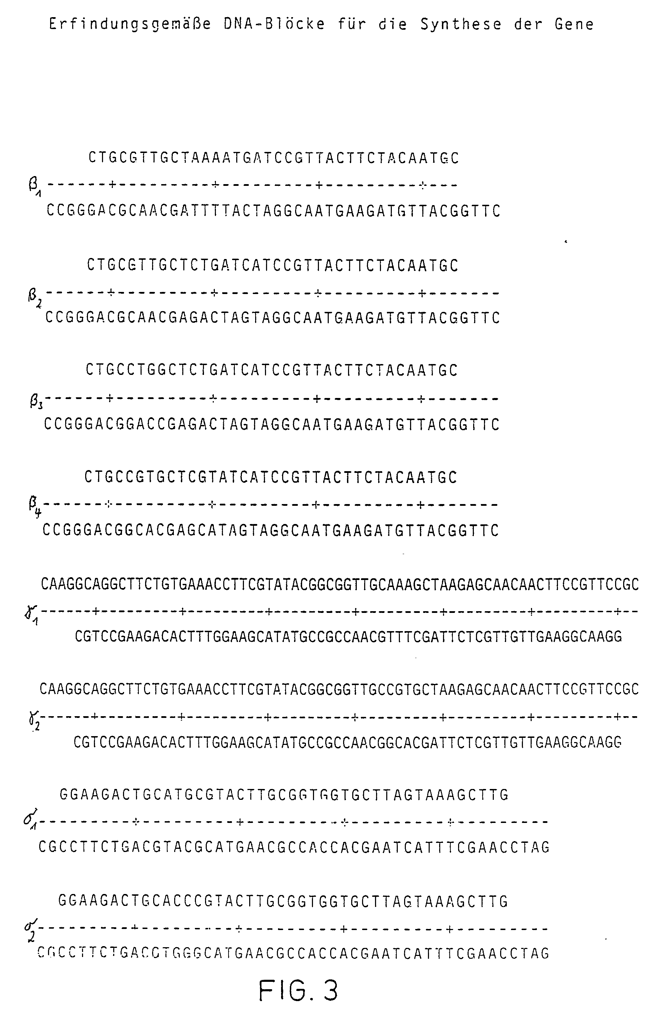

Gegenstand dieser Erfindung sind insbesondere auch Desoxyribonucleinsäuren der folgenden, in Abb. 3 zusammengestellten Sequenzen.

Ebenso werden funktionelle Äquivalente der synthetischen Desoxyribonucleinsäure-Sequenzen mit anderen "codon usages" beansprucht, bei deren Translation die erfindungsgemäßen Inhibitoren erhalten werden.

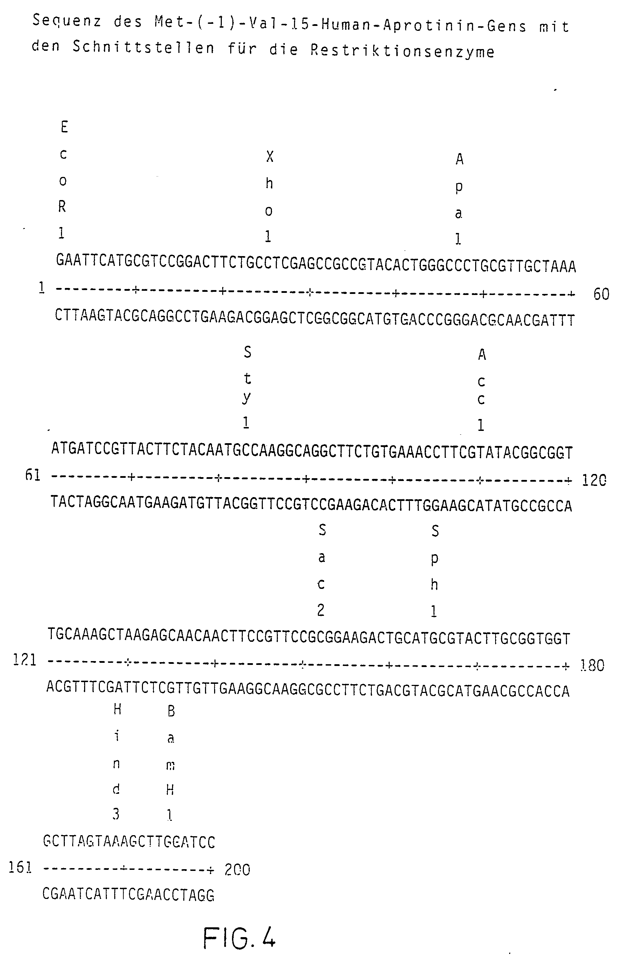

In Abb. 4 ist die für das humane Aprotinin mit Val in Position 15 codierende DNA-Region wiedergegeben. Die Synthese dieses Gens erfolgte durch Ligation von bereits bekannten - vgl. E.-A. Auerswald u.a. EP 87 103 942.3 -bzw. in Abb. 3 aufgelisteten DNA-Sequenzblöcken. Aus dieser Abbildung sind auch die 4 das Gen konstituierenden Sequenzblöcke ersichtlich.

Die DNA-Blöcke sind, wie Abb. 4 zeigt, durch singulare Schnittstellen der Restriktionsenzyme: EcoR I, Apa I, Sty I und Sac II begrenzt. Sie sind daher bequem austauschbar. Die für die anderen beanspruchten Inhibitoren benötigten DNA-Varianten wurden entweder synthetisch oder durch "site-directed Mutagenesis" erhalten.

In Tabelle 2 ist beispielhaft die Kombination der DNA-Blöcke aus Abb. 3 für einige erfindungsgemäße Inhibitoren zusammengestellt.

**Tabelle 2**

Kombination der zur Synthese der für die "Coding-Regionen" der erfindungsgemäßen Inhibitoren benötigten bekannten bzw. in Abb. 3 aufgelisteten synthetischen DNA-Blöcke.

| Inhibitor | Gensynthese-beispiel | Kombination der DNA-Blöcke |
|---|---|---|
| Val-15-Human-Aprotinin | 1 | $\alpha_1^* - \beta_1 - \gamma_1 - \delta_1$ |
| Val-15-Leu-17-Ile-18-Human Aprotinin | 2 | $\alpha_1 - \beta_2 - \gamma_1 - \delta_1$ |
| Val-15-Leu-17-Ile-18-Thr-52-Human-Aprotinin | 3 | $\alpha_1 - \beta_2 - \gamma_1 - \delta_2$ |
| Leu-15-Leu-17-Ile-18-Thr-52-Human-Aprotinin | 4 | $\alpha_1 - \beta_3 - \gamma_1 - \delta_2$ |
| Arg-15-Arg-17-Ile-18-Arg-39-Glu-52-Human-Aprotinin | 5 | $\alpha_1 - \beta_4 - \gamma_2 - \delta_3$ |

```
                      CTGCGTTGCTAAAATGATCCGTTACTTCTACAATGC
*)  α-Block:  -----+---------+---------+---------+-------
                      CCGGGACGCAACGATTTTACTAGGCAATGAAGATGTTACGGTTC
```

und $\delta_3$-Block:

```
        GGAAGACTGCGAACGTACTTGCGGTGGTGCTTAGTAAAGCTTGGATCC
        ---------+---------+---------+---------+---------+
        CGCCTTCTGACGCTTGCATGAACGCCACCACGAATCATTTCGAACCTAGG
```

sind beschrieben bei: E.-A. Auerswald u.a., EP 87 103 942.3 vom 26.03.86

Je nach der Natur des Expressionssystems können die erfindungsgemäßen Gene für die Expression der beanspruchten Proteinaseinhibitoren zusätzlich ein Start-Codon für Met oder eine Nucleotidsequenz vorgeschaltet haben, die für eine Leadersequenz codiert.

Ein weiterer Gegenstand dieser Erfindung sind auch von Plasmiden abgeleitete Expressionsvektoren, in die die erfindungsgemäßen für die beanspruchten Proteinaseinhibitoren codierenden Gene inseriert sind und die für die Transformation in geeignete Wirtsstämme verwendet werden.

Es ist bereits bekannt geworden, daß in bakteriellen Wirtsstämmen eine erhöhte Expression an Säugetierproteinen erhalten wird, wenn das für das gewünschte heterologe Protein codierende Gen mit einem für ein hoch exprimierbares mikrobielles Protein codierendes Gen fusioniert wird. Die gewünschten unfusionierten Proteine sind aus den Fusionsproteinen durch selektive enzymatische vorzugsweise jedoch durch chemische Spaltung zugänglich, wenn die Verknüpfung der Fusionspartner über eine Aminosäure (Met, Trp) erfolgt, an deren C-Terminus mit geeigneten Reagentien eine selektive Spaltung durchgeführt werden kann. Voraussetzung ist, daß diese Aminosäure nicht auch Bestandteil der erfindungsgemäßen Proteinaseinhibitoren ist.

Bevorzugte Fusionspartner für die beanspruchten Proteinaseinhibitoren können sekretorische Signalsequenzen von mikrobiellen Proteinen sein, wie z.B. omp A -Ghray-ed u.a. EMBO Journal 3, 2437-2442 (1984) - oder pho A - Gray u.a., Gene 39, 247-254 (1985) -.

Die Fusionsproteine werden dann während der Fermentation in das Periplasma oder Wuchsmedium sezerniert und nach an sich bekannten Verfahren aus den Kulturfiltraten isoliert. Bei der Wahl geeigneter Fusionspartner für sekretorische Systeme werden die Proteinaseinhibitoren in die Fusionsproteinen mit nativer Struktur sezerniert. Eine Renaturierung nach der Bromcyan-Spaltung - E. Gross und B. Witkop, J. Amer. Chem. Soc. 83, 1510-1511 (1961) -entfällt für den Fall einer korrekten Prozessierung beim Membrandurchtritt. Die erfindungsgemäßen Proteinaseinhibitoren werden aus den Reaktionsgemischen nach an sich bekannten Verfahren isoliert und gereinigt.

Eine alternative Möglichkeit der Gen-Expression ist im Falle der erfindungsgmeäßen Proteinaseinhibitoren realisierbar, wenn die Fusionsproteine in der Zelle unlöslich in Form von "Inclusion Bodies" ausgeschieden werden. Solche Einschlüsse von unlöslichen Fusionsproteinen können erzeugt werden z.B. bei der Fusionierung der Inhibitorgene mit einer DNA-Teilsequenz der DNA-Polymerase des Bacteriophagen MS2 - E. Remault, P. Stanssens und W. Fiers, Gene 15, 81 (1981) - oder mit dem lacZ-Gen von Escherichia coli - E.-A. Auerswald u.a., Patentanmeldung EP 87 103 942.3 vom 26.03.1986 - und auch bei der Fusion der Inhibitorgene mit einer Teilsequenz des cII-Gens der Bacteriophagen Lambda - K. Nagai und H.C. Thorgersen, Nature 309, 810 (1984) -.

Die Produktion der erfindungsgemäßen Proteinaseinhibitoren über Einschlußkörper umfaßt die folgenden Schritte:

1. Anzucht und Kultivierung des Wirtsstammes unter geeigneten Bedingungen;
2. Isolierung der Einschlußkörper aus den Wirtszellen;
3. Aufreinigung der Fusionsproteine;
4. Spaltung der Fusionsproteine;
5. Renaturierung der Inhibitoren;
6. Isolierung und Reinigung der Inhibitoren.

Die einzelnen Schritte sind in den Beispielen ausführlich beschrieben.

Für die Reinigung der löslichen Fusionsproteine kann es vorteilhaft sein, in den abspaltbaren Fusionspartner gehäuft saure oder basische Aminosäuren einzubauen, so daß die Abtrennung durch Ionenaustauschchromatographie erleichtert wird.

Die Endreinigung der erfindungsgemäßen Proteinaseinhibitoren erfolgt nach an sich bekannten Verfahren, wie z.B. Gelfiltration, Ionenaustauschchromatographie oder Affinitätschromatographie und Elektrophorese.

Derzeitig kennt man eine große Zahl von verschiedenen Mikroorganismen, die für die Transformation geeignet sind: nämlich einzellige Organismen, die in Kulturen oder Fermentationsbrühen gezüchtet werden können. Bevorzugte Organismen zur Transformation schließen Bakterien, Hefen und Pilze ein.

Der für die hier beschriebenen Arbeiten verwendete spezielle Organismus war E. coli RR1ΔM15, der bei der American Type Culture Collection unter der ATCC Nr. 35102 hinterlegt wurde. Es können auch andere geeignete E. coli Stämme verwendet werden.

E. coli RRIΔM 15 transformiert mit dem Plasmid pRK 151.1.8 (Val-15-Leu-17-Ile-18-Thr-52-Human-Aprotinin, siehe Beispiel 3) wurde unter der Nummer DSM 4160. E. coli RRIΔM 15 transformiert mit dem Plasmid pRK 155.1.4 (Leu-15-Leu-17-Ile-18-Thr-52-Human Aprotinin) wurde unter der Nummer DSM 4161 am 26. 6. 1987 bei der Deutschen Sammlung von Mikroorganismen, Grisebachstr. 8, D-3400 Göttingen, hinterlegt.

Die vorliegende Erfindung umfaßt pharmazeutische Zubereitungen, die außer nicht-toxischen, inerten, pharmazeutisch geeigneten Exzipienten eine oder mehrere Verbindungen gemäß der Erfindung umfassen oder aus einer oder mehreren aktiven Verbindungen gemäß der Erfindung bestehen, sowie Verfahren zur Herstellung dieser Zubereitungen.

Die vorliegende Erfindung umfaßt auch pharmazeutische Zubereitungen in Dosiseinheiten. Dies bedeutet, daß die Zubereitungen in Form individueller Teile vorliegen, z.B. als Tabletten, beschichtete Tabletten, Kapseln, Pillen, Suppositorien und Ampullen, deren Gehalt an Wirkstoff einer Fraktion oder einem Vielfachen einer individuellen Dosis entspricht. Die Dosiseinheiten können z.B. enthalten: ein, zwei, drei oder vier individuelle Dosen oder eine Hälfte, ein Drittel oder ein Viertel einer individuellen Dosis. Eine individuelle Dosis enthält vorzugsweise die Menge an Wirkstoff, die bei einer Verabreichung gegeben wird, und die gewöhnlich dem Ganzen, der Hälfte oder einem Drittel oder einem Viertel der Tagesdosis entspricht.

Unter nicht-toxischen, inerten, pharmazeutisch geeigneten Exzipienten sind zu verstehen: feste, halbfeste oder flüssige Verdünner, Füller und Formulierungshilfsmittel aller Art.

Tabletten, beschichtete Tabletten, Kapseln, Pillen, Granalien, Suppositorien, Lösungen, Suspensionen und Emulsionen, Pasten, Salben, Gele, Cremes, Lotionen, Pulver und Sprays können als bevorzugte pharmazeutische Zubereitungen genannt werden.

Tabletten, beschichtete Tabletten, Kapseln, Pillen und Granalien können den Wirkstoff oder die Wirkstoffe zusammen mit üblichen Exzipienten, wie (a) Füller und Streckmittel, z.B. Stärken, Lactose, Sucrose, Glucose, Mannit und Siliziumdioxid, (b) Bindemittel, z.B. Carboxymethylcellulose, Alginate, Gelatine und Polyvinylpyrrolidon, (c) anfeuchtende Mittel, z.B. Glycerin, (d) disintegrierende Mittel, z.B. Agar-Agar, Kalziumcarbonat und Natriumcarbonat, (e) Lösungsverzögerer, z.B. Paraffin und (f) Absorptionsbeschleuniger, z.B. quaternäre Ammoniumverbindungen, (g) Benetzungsmittel, z.B. Cetylalkohol und Glycerinmonostearat, (h) Absorbentien, z.B. Kaolin und Bentonit und (i) Schmiermittel, z.B. Talk, Kalzium- und Magnesiumstearat und feste Polyethylenglykole, oder Gemische der unter (a) bis (i) aufgeführten Substanzen, enthalten.

Die Tabletten, beschichteten Tabletten, Kapseln, Pillen und Granalien können mit üblichen Überzügen und Umhüllungen versehen werden, die gegebenenfalls Opazifizierungsmittel enthalten; sie können eine Zusammensetzung aufweisen, so daß die Freisetzung des Wirkstoffes oder der Wirkstoffe nur, oder vorzugsweise, in einem bestimmten Teil des Intestinaltraktes, gegebenenfalls in verzögerter Form, erfolgt, wobei Beispiele für geeignete Zubereitungen zum Einbetten polymere Substanzen und Wachse bilden.

Die aktive Verbindung oder Verbindungen, gegebenenfalls zusammen mit einem oder mehreren der vorstehend genannten Exzipienten, kann bzw. können auch in mikroverkapselter Form vorliegen.

Suppositorien können außer dem Wirkstoff bzw. den Wirkstoffen enthalten: übliche wasserlösliche oder wasserunlösliche Exzipienten, z.B. Polyethylenglykole, Fette, z.B. Kakaofett und höhere Ester (z.B. $C_{14}$-Alkohol mit $C_{16}$-Fettsäure) oder Gemische dieser Substanzen.

Salben, Pasten, Cremes und Gele können zusätzlich zu dem Wirkstoff oder den Wirkstoffen übliche Exzipienten enthalten, z.B. tierische und pflanzliche Fette, Wachse, Paraffine, Stärke, Tragacanth, Cellulosederivate, Polyethylenglykole, Silikone, Bentonite, Siliziumdioxid, Talk und Zinkoxid, oder Gemische dieser Substanzen.

Pulver und Sprays können außer dem Wirkstoff oder den Wirkstoffen übliche Exzipienten enthalten, z.B.: Lactose, Talk, Siliziumdioxid, Aluminiumhydroxid, Kalziumsilikat und Polyamidpulver, oder Gemische dieser Substanzen. Sprays können außerdem die üblichen Treibmittel enthalten, z.B. Chlorofluorkohlenwasserstoffe.

Lösungen und Emulsionen können außer dem Wirkstoff oder den Wirkstoffen übliche Exzipienten umfassen, wie Lösungsmittel, solubilisierende Agentien und Emulgiermittel, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsamenöl, Erdnußöl, Maiskeimöl, Olivenöl, Rizinusöl und Sesamöl, Glycerin, Glycerinformal, Tetrahydrofurfurylalkohol, Polyethylenglykole und Fettsäureester von Sorbitan, oder Gemische dieser Substanzen.

Zur parenteralen Verabreichung können die Lösungen und Emulsionen auch in einer sterilen Form vorliegen, die mit Blut isoton ist.

Suspensionen können zusätzlich zum Wirkstoff oder den Wirkstoffen übliche Exzipienten enthalten, wie flüssige Verdünner, z.B. Wasser, Ethylalkohol oder Propylenglykol, Suspendierungsmittel, z.B. ethoxylierte Isostearylalkohole, Polyoxyethylensorbit und Sorbitanester, mikrokristalline Cellulose, Aluminium-metahydroxid, Bentonit, Agar-Agar und Tragacanth, oder Gemische dieser Substanzen.

Die genannten Formulierungsformen können auch enthalten: Farbstoffe, Konservierungsmittel und Additive, die den Geruch oder Geschmack verbessern, z.B. Pfefferminzöl und Eukalyptusöl, und Süßungsmittel, z.B. Saccharin.

Die therapeutisch wirksamen Verbindungen sollten in den vorstehend genannten pharmazeutischen Zubereitungen vorzugsweise in einer Konzentration von 0,1 bis 99,5 Gew.-%, vorzugsweise von ca. 0,5 bis 95 Gew.-%, bezogen auf das Gesamtgemisch, vorliegen.

Die vorstehend genannten pharmazeutischen Zubereitungen können zusätzlich zu den Verbindungen gemäß der Erfindung auch andere pharmazeutische Wirkstoffe enthalten.

Die vorstehend genannten pharmazeutischen Zubereitungen werden in üblicher Weise nach bekannten Verfahren hergestellt, z.B. durch Vermischen des Wirkstoffes oder der Wirkstoffe mit dem Exzipienten oder den Exzipienten.

Die Wirkstoffe oder die pharmazeutischen Zubereitungen können lokal, oral, parenteral, intraperitoneal und/oder rektal verabreicht werden, vorzugsweise erfolgt dies oral oder parenteral, wie z.B. intravenös oder intramuskulär.

Im allgemeinen hat es sich sowohl in der Humanmedizin als auch in der Tiermidizin als vorteilhaft erwiesen, den Wirkstoff oder die Wirkstoffe gemäß der Erfindung in den Gesamtmengen von ca. 0,5 bis ca. 500, vorzugsweise von 4 bis 100 mg/kg Körpergewicht alle 24 Stunden, gegebenenfalls in Form von mehreren individuellen Verabreichungen, zu geben, um die besten Ergebnisse zu erzielen. Eine individuelle Verabreichung enthält den Wirkstoff oder die Wirkstoffe gemäß der Erfindung vorzugsweise in Mengen von ca. 1 bis ca. 250, insbesondere 3 bis 60 mg/kg Körpergewicht. Es kann jedoch erforderlich sein, von der genannten Dosis abzuweichen, insbesondere in Abhängigkeit von der Natur und dem Körpergewicht des zu behandelnden Individuums, der Natur und Schwere der Krankheit, der Art der Zubereitung und der Verabreichung der Medizin, und der Zeit oder dem Intervall, über den die Verabreichung stattfindet.

So kann es in einigen Fällen genügen, mit weniger als der vorstehend genannten Menge an Wirkstoff auszukommen, während in anderen Fällen die vorstehend genannte Menge an Wirkstoff überschritten werden muß. Die speziell erforderliche optimale Dosis und die Verabreichungsart der Wirkstoffe kann in einfacher Weise vom Fachmann auf der Basis seines Fachwissens entschieden werden.

Die erfindungsgemäßen Wirkstoffe sind sehr gut zur Behandlung von akuten Entzündungen, wie rheumatoider Arthritis, hereditäres angioneurotisches Ödem, Pneumonie, Pankreatitis und von Schockzuständen einsetzbar. Weiterhin haben die Arg-15-Homologen eine protektive Funktion bei Dialysen mit Hilfe von künstlichen Membranen und allgemein bei der extrakorporalen Zirkulation.

## Material und Methoden

### A. Konstruktion der synthetischen Gene und der rekombinanten Klonierungs- und Expressionsvektoren

#### Materialien:

#### 1. Reagentien

Reagentien, die für die Herstellung von Agarose-Gelen, Polyacrylamid-Gelen, Reaktionspuffern und Kulturmedien erforderlich waren, wurden von Boehringer (Mannheim) Difco, Merck, Serva, Sigma und Biorad bezogen.

#### 2. Enzyme

Die Enzyme für molekularbiologische Klonierungsarbeiten wurden von Boehringen (Mannheim), Biolabs, und BRL bezogen.

#### 3. DNA

Plasmid- und Phagen-DNA sowie DNA-Adapter für die Gen- und Vektorenkonstruktionen wurden bis auf nachfolgende Ausnahmen von Boehringer, Biolabs, BRL und Pharmacia bezogen.

#### Ausnahmen:

Plasmid pUR 278: U. Rüther und B. Müller-Hill, EMBO Journal 2, 1791-1794 (1983); Plasmid pEx 31.b: (Strebel et. al, J. of Virology (1986) 57, 983-991).

4. E.coli-Stämme

E.coli RR1ΔM15 (ATCC Nr. 35102): U. Rüther, Nucleic Acids Res. 10, 5765-5772 (1982).
E.coli 537-W6, rex: W. Fiers, Universität Gent, Belgien - E. Remault u.a. (1981), Gene 15, 81-93 -.

5. Medien

Die Rezepturen für LB-, M9s- und Kappa 1776-Medium entsprechen den Angaben in: T. Maniatis u.a. - Molecular Cloning, Cold Spring Harbor, USA (1982) -. Für Agar-Platten wurde den Medien 15 g Bacto-Agar hinzugefügt.

6. Radioisotope

L-[$^{35}$S]-Methionin, Adenosin-5$^{\prime}$-α[$^{35}$S]-thiotriphosphat und [$^{14}$C]Protein Molekulargewichtsmarker für die Gelelektrophoresen wurden von Amersham-Buchler bezogen.

7. Antibiotika

Für die Herstellung von Selektionsmedien wurden folgende Antibiotika verwendet: Chloramphenicol und Kanamycin von Boehringer; Ampicillin und Tetracyclin von Serva.

Methoden

Die Routine-Techniken für molekulare Klonierungsexperimente, wie Isolierung und Aufreinigung von Plasmid-DNA, Isolierung von DNA-Fragmenten, Restriktionsanalysen, Auftrennung von DNA-Fragmenten auf Agarose- und Acrylamidgelen, Transformation von Bakterien etc. sind beschrieben in: T. Maniatis u.a., Molecular Cloning, Cold Spring Harbor (1982).

8. Chemische Synthese von DNA-Oligonukleotiden

Die für Konstruktion der Proteinaseinhibitorgene benötigten DNA-Oligonukleotide wurden mit einer automatischen DNA-Synthese-Maschine von Applied Biosystems, Model 380, synthetisiert. Die Aufreinigung der einzelsträngigen DNA-Oligonukleotide erfolgte über HPLC-Säulen oder durch präparative Acrylamidgelelektrophorese. Es wurden jeweils 0,5 bis 5,0 OD$_{260nm}$-Einheiten der einzelnen Oligomeren isoliert.

9. DNA-Sequenzierung

Die zu analysierenden DNA-Fragmente wurden entweder in M13 Phagen-Vektoren oder Plasmid-Vektoren der pUC-Serie kloniert und die DNA-Sequenz anschließend überprüft. Die Sequenzierung der DNA erfolgte nach der Vorschrift von N. Hattori und S. Sakaki - Analyt. Biochem. 152, 232-238 (1986) - und einem Se quenzierungshandbuch von Boehringer Mannheim -Leitfaden zum schnellen und einfachen Plasmid-Sequenzieren (1986) -.

10. Standard Expression der humanen Aprotioninvarianten

Zur Expression der humanen Aprotininvarianten wurden sowohl Sekretionsvektoren als auch Expressionsvektoren zur Produktion von cytoplasmatischen Einschlußkörpern verwendet.
Die korrekte Insertion der humanen Aprotinin-Genvarianten in die verschiedenen Expressionssysteme wurde durch DNA-Sequenzierung kontrolliert.
Die rekombinanten Sekretionsvektoren und der lacZ-Vektor wurden in den E.coli Stamm RR1ΔM15 eingeschleust. Der P-MS2-Polymerase-Vektor wurde in den E.coli Stamm 537 eingeschleust.

a) Induktion der Sekretionsvektoren und des lacZ-Vektors

Frische Übernachtkulturen der transformierten E.coli Stämme wurden 1:100 in 10 ml LB-Medium (für analytische Zwecke) bzw. in 1 l LB Medium (für präparative Zwecke) verdünnt. Dem Medium wurde 100 ug/ml Ampicillin zugesetzt.

Die Kulturen wurden bei 37°C und 200 UpM bis zu einer $OD_{550nm}$ = 1,0 in Erlenmeyerkolben inkubiert. Die Induktion der Expressionssysteme (lac-Promoter) erfolgte durch Zugabe von 1 mM IPTG (Isopropylthiogalactosid).

Nach 16 bis 24 Stunden Induktion bei 37°C wurden die Kulturen mittels Zentrifugation geerntet (10 Minuten; 15 bis 20.000 g; 4°C). Bei Verwendung der Sekretionsvektoren wurden die Kulturüberstände anhand der enzyminhinitorischen Aktivität auf den Gehalt der sezernierten humanen Aprotininvarianten getestet (s. Methodenteil B, 10).

Die mit $\beta$-Galaktosidase als Fusionspartner exprimierten humanen Aprotininvarianten wurden nach Aufschluß der Bakterien mit SDS-Polyacrylamid-Gelelektrophoresen - U.K. Laemmli, Nature 277, 680 (1970) - nachgewiesen. Hierzu wurden pro Gelspur 1 x 10^9 Zellen in 40 $\mu$l 0,1 M Trispuffer pH 8.0, der 0.01 M EDTA enthält suspendiert und nach Zugabe von 10 $\mu$l SDS-Probenpuffer (0,3 M Tris-HCl, pH 8.8; 50 % Glycerin, 5 % SDS. 25 % $\beta$-Mercaptoethanol) 3 Minuten gekocht. Nach der Elektrophorese wurden die Proteinbanden mit Coomassie Blau eingefärbt und der prozentuale Anteil an Fusionsprotein mit einem Densitometer bestimmt.

b) Induktion des $\lambda P_L$-MS2-Polymerase Vektors

Die Expression der humanen Aprotininvarianten als Fusionsproteine mit einer Teilsequenz der DNA-Polymerase des Phagen MS2 erfolgte mit einem pEx-Vektor (Strebel et. al, J. of Virology (1986) 57,983-991) unter Kontrolle des $P_L$-Promoters des Phagen Lambda.

Die Anzucht der transformierten E.coli Stämme (Derivate von Ec 537) erfolgte in 10 ml bzw. 1 l LB-Medium mit 100 $\mu$g/ml Ampicillin bei 200 UpM und 28°C. Bei einer Wuchsdichte von $OD_{550nm}$ = 1,0 wurde die Temperatur auf 42°C erhöht (Hitzeinduktion) und die Kulturen für weitere 3 Stunden bei 200 UpM inkubiert.

Die Zellen wurden durch Zentrifugation geerntet (s.o.), in SDS-Probenpuffer aufgeschlossen und Aliquots auf SDS-Polyacrylamidgelen durch Elektrophorese aufgetrennt.

Die in Form von Einschlußkörpern akkumulierten Fusionsproteine wurden durch Färbung der Gele mit Coomassie Blau nachgewiesen und der prozentuale Anteil der Fusionsproteine densitometrisch gemessen.

B) Isolierung und Charakterisierung der erfindungsgemäßen Proteinaseinhibitoren

Materialien

1. Enzyme

Humane Leukozytenelastase - SE 563 - und humanes Cathepsin G - SG 45 - wurden von Elastin Products Company Inc., P.O. Box 147. Pacific, Ma. 63069, USA bezogen; Trypsin (Rind) von E. Merck, Darmstadt. Humanes Plasmakallikrein - 80-13-1101 - stammte von der Fa. Protogen AG, Weidenmattweg 4, Postfach, CH-4448 Läufelfingen, Schweiz. Humanes Harnkallikrein wude von Dr. R. Geiger, Abtlg. f. Klinische Chemie und Klinische Biochemie in der Chirurgischen Klinik, Universität München, Nußbaumstr. 20, 8000 München 2, überlassen, humanes anionisches und kationisches Trypsin von Dr. K. Ohlsson, Dept. Clin. Chemistry and Surgery, Malmö General Hospital. S-21401 Malmö, Schweden.

## 2. Substrate

Suc-Ala-Ala-Val-pNA und MeOSuc-Ala-Ala-Pro-Val-pNA (HGE) sowie MeOSuc-Ala-Ala-Pro-Met-pNA (HCG) wurden von der Fa. Bachem, Feinchemikalien AG, Hauptstr. 144, CH-4416 Bubendorf, Schweiz bezogen;

D-Pro-Phe-Arg-pNAx2HCl = S-2302 (Plasmakallikrein),
D-Val-Leu-Arg-pNAx2HCl = S-2260 (Organkallikrein)
und Pyr-Gly-Arg-pNAxHCl = S-2444 (Trypsin) wurden von der Deutschen Kabi GmbH, Levelingstr. 18, D-8000 München 80 gekauft.

## 3. Anti-Aprotinin Antikörper

Anti-Aprotinin Antikörper wurden im Kaninchen erzeugt und aus dem Serum isoliert, wie von A. Eben und E. Truscheit beschrieben - vgl. dazu G.L. Haberland in: Synthetic and Natural Proteinase Inhibitors: Basic and Clinical Aspects Intern. Symp., Tokio 20.11.1967. Japanese Society Medical Sci. 1967, 47-55 - zur weiteren Reinigung wurden die Antikörper über eine mit auf Sepharose 4B-Cl immobilisiertem Aprotinin beschickten Säule chromatographiert. Dazu wurde die Säule mit Saline-Phosphatpuffer gewaschen, anschließend mit 200 ml 0,2 M Kaliumcyanat und nochmals 200 ml Saline-Phosphatpuffer. Die Desorption wurde mit 0,2 M Glycin-Salzsäurepuffer pH 2,4 durchgeführt. Die die Antikörper enthaltenden Fraktionen wurden sofort mit gesättigter Tris-Lösung neutralisiert und über Nacht bei 4°C gegen 50 Volumina 0,1 M Natriumhydrogencarbonatlösung dialysiert.

## Methoden

## 4. Immobilisierung der Kaninchen Anti-Aprotinin Antikörper auf Sepharose Cl-4B

200 g Sepharose 4B wurden mit Bromcyan nach dem Verfahren von J. Kohn und M. Wilchek - Appl. Biochem. Biotechnol. 9, 285, (1984) - aktiviert und anschließend mit 2 g Anti-Aprotinin Antikörpern umgesetzt.

## 5. HPLC

Präparative und analytische HPLC wurde mit geeigneten Säulen durchgeführt, wie bei den einzelnen Beispielen beschrieben.

## 6. Aminosäuresequenzbestimmungen

Für die Bestimmung der Aminosäuresequenzen der erfindungsgemäßen Proteinaseinhibitoren wurden 0,5 bis 2 nMol Substanz auf ein mit Polybren® vorbehandeltes Glasfaserfilter geladen und die Sequenzanalyse mit dem Gasphasen Protein Sequenzer - Fa. Applied Biosystems 470 - durchgeführt. Die in jedem Cyclus freigesetzten Aminosäure-phenylthiohydantoine wurden mittels HPLC an einer Zorbax CN-Säule durch isokratische Elution nach Beyreuther - K. Beyreuther, B. Biesler, J. Bovens, R. Dildrop, K. Neifer, K. Stüber, S. Zais, R. Ehring und P. Zabel in: Modern Methods in Protein Chemistry S. 303-325 (1983), Walter de Gruyter, Berlin -identifiziert und quantifiziert.

## 7. Bestimmung der quantitativen Aminosäurezusammensetzung

Jeweils ca. 1 nMol Inhibitor wurde nach J.T. Potts jr. - Anal. Biochem. 131, 1-15 (1969) - mit 200 µl konstantsiedender Salzsäure, die 0,05 % Mercaptoethanol enthielt, in einem abgeschmolzenen evakuierten Pyrexröhrchen 22 Stunden auf 110°C erhitzt. Die Eindampfrückstände wurden in 150 µl 0,2 M Natriumcitratpuffer pH 2,2 aufgenommen und unlösliche Anteile durch Filtration abgetrennt. Die in den Hydrolysaten vorliegenden Aminosäuren wurden in einem Biotronic LC 5000 Aminosäureanalysator getrennt, der mit einem Fluoreszensdetektor und einem Shimadzu CR 2AC-Integrator ausgerüstet war. Die Quantifizierung erfolgte nach Derivatisierung mit o-Phthaldialdehyd, wie von J.R. Benson und P.E. Hare - Proc. Nat. Acad. Sci. 72, 619-622 (1975) -beschrieben. Die quantitative Aminosäurezusammensetzung einiger erfindungsgemäßen Inhibitoren ist in Tabelle 3 angegeben.

**Tabelle 3**  Quantitative Aminosäurezusammensetzung einiger erfindungsgemäßer Proteinaseinhibitoren

| Amino-säure | Val-15-HA[1] ber. | Val-15-HA[1] gef. | Val-15-Leu-17-Ile-18-HA ber. | Val-15-Leu-17-Ile-18-HA gef. | Val-15-Leu-17-Ile-18-Thr-52-HA ber. | Val-15-Leu-17-Ile-18-Thr-52-HA gef. | Leu-15-Leu-17-Ile-18-Thr-52-HA ber. | Leu-15-Leu-17-Ile-18-Thr-52-HA gef. | Arg-15-Arg-17-Ile-18-Arg-39-Glu-52-HA ber. | Arg-15-Arg-17-Ile-18-Arg-39-Glu-52-HA gef. |
|---|---|---|---|---|---|---|---|---|---|---|
| Asp | 5 | 5,15 | 5 | 5,05 | 5 | 5,09 | 5 | 5,11 | 5 | 5,13 |
| Thr | 3 | 2,89 | 3 | 2,78 | 4 | 3,80 | 4 | 3,79 | 3 | 2,83 |
| Ser | 2 | 1,70 | 1 | 1,65 | 2 | 1,74 | 2 | 1,80 | 2 | 1,74 |
| Glu | 3 | 3,20 | 3 | 3,15 | 3 | 3,25 | 3 | 3,18 | 4 | 4,30 |
| Gly | 6 | 6,05 | 6 | 6,01 | 6 | 6,08 | 6 | 6,10 | 6 | 5,89 |
| Ala | 6 | 6,16 | 6 | 6,20 | 6 | 6,00 | 6 | 6,00 | 6 | 6,00 |
| Val | 2 | 2,04 | 2 | 2,03 | 2 | 2,05 | 1 | 1,05 | 1 | 1,03 |
| Met | 2 | 1,80 | 1 | 0,92 | 0 | - | 0 | - | 0 | - |
| Ile | 1 | 0,93 | 2 | 1,34 | 2 | 1,29 | 2 | 1,30 | 2 | 1,31 |
| Leu | 1 | 1,00 | 2 | 2,00 | 2 | 2,03 | 3 | 2,95 | 1 | 0,96 |
| Tyr | 4 | 3,86 | 4 | 3,90 | 4 | 3,74 | 4 | 3,69 | 4 | 3,78 |
| Phe | 5 | 4,95 | 5 | 5,10 | 5 | 4,89 | 5 | 4,93 | 5 | 5,07 |
| Lys | 4 | 3,89 | 3 | 2,92 | 3 | 3,04 | 3 | 2,94 | 4 | 3,82 |
| Arg | 4 | 3,89 | 4 | 3,95 | 4 | 4,05 | 4 | 4,10 | 5 | 4,89 |

[1] HA = Human-Aprotinin

[2] Bezugsaminosäure: Leu bzw. Ala

EP 0 297 362 A2

## 8. Polyacrylamidgelektrophoresen

Die SDS-Gelelektrophoresen wurden nach Laemmli - U.K. Laemmli, Nature 227, 680 (1970) - durchgeführt. Die Proteinbanden wurden angefärbt, wie von J. L. Stephano, M. Gould und L. Rojas-Galicia - Anal. Biochem. 152, 308 (1986) - beschrieben.

## 9. Renaturierung der inaktiven Inhibitoren nach der Bromcyan-Spaltung

Die Renaturierung der nach Bromcyan-Spaltung erhaltenen inaktiven Inhibitoren erfolgte nach dem von Creighton erstmals für Aprotinin beschriebenen Verfahren - T.E. Creighton, in: UCLA Symposia on Molecular and Cellular Biology 39, 249-257 (1986), D.L. Oxender, Ed., A.R. Liss, Inc., New York - am Ionenaustauscher unter Verwendung eines Harnstoffgradienten mit einigen Abänderungen. Die exakten Bedingungen sind bei der Beschreibung der einzelnen Beispiele angegeben.

## Enzymteste

## 10. Bestimmung des Inhibitorgehaltes in mikrobiellen Proben, die die Inhibitoren entweder korrekt prozessiert oder als lösliche Minifusionsproteine enthalten

Eine Probe der Fermentationsbrühe wurde durch Zentrifugieren geklärt und die inhibitorische Aktivität im Überstand und in den Zellen wie folgt bestimmt:

## a) Kulturfiltrat:

1 ml Kulturfiltrat wurde mit 10 μl einer 5 %igen wäßrigen Lösung von Tween 80 und 40 μl Perchlorsäure (70 %ig) unter gutem Durchmischen versetzt. Man hielt die Mischung 30 Minuten bei Raumtemperatur und trennte den gebildeten Niederschlag durch Zentrifugieren ab. Die überstehende klare Lösung wurde durch Zugabe von 180 μl gesättigter Trislösung neutralisiert.

Zu 420 μl 0.2 M Tris-Salzsäure-Puffer pH 8.0, der 0.05 % Natriumazid und 0.1 % Tween 80 enthält, fügte man 8 μl einer frisch hergestellten Lösung von humaner Leukozytenelastase -erhalten durch einhundertfache Verdünnung der 1 mg Enzym je ml 0.05 M Natriumacetatpuffer pH 5.5 - Ethylenglykol (1:1) enthaltenden Lösung mit Testpuffer - und in einer Konzentrationsreihe die gewünschte Volumina des neutralisierten Fällungsüberstandes. Dann wurde in jeder Probe das Volumen mit Testpuffer auf 550 μl ergänzt, wobei die 100 % - Probe nur Testpuffer enthielt. Nach 30 Minuten Vorinkubation bei Raumtemperatur wurde mit jeweils 100 μl einer Mischung von Testpuffer und 6.5 μl einer 0.1 M Lösung von MeOSuc-Ala-Ala-Pro-Val-pNA versetzt und der durch die enzymatische Freisetzung von p-Nitroanilin bedingte Extinktionsanstieg bei 405 nm bestimmt. Die prozentuale Inhibition errechnet sich nach:

$$\% \text{ Inhibition} = 100 \times \left[1 - \frac{\text{OD mit Inhibitorzusatz}}{\text{OD ohne Inhibitorzusatz}}\right]$$

Die quantitative Ermittlung der Inhibitorgehalte erfolgte unter Verwendung von mit rekombinanten Val-15-Glu-52-Aprotinin erstellten Eichkurven.

b) <u>Zellen:</u>

Die beim Zentrifugieren der Kulturbrühen erhaltenen Zellen wurden in 1/10-Originalvolumen 50 mM Tris-HCl-Puffer pH 7,5 aufgeschlämmt, der 0,05 %ig an Tween 80 war.

Die Zellen wurden unter Verwendung eines Ultraschallgerätes - Branson Sonifier, Cell Disruptor B 15 - bei 4°C aufgeschlossen (400 Watt, 1 Minute Beschallung je ml Probenvolumen). Nach der Fällung der endogenen Inhibitoren mit Perchlorsäure in der oben beschriebenen Weise, wurde der Inhibitorgehalt analog bestimmt.

<u>11. Ermittlung der Hemmspezifität der Inhibitoren</u>

Die Bedingungen für die Testung der Reinsubstanzen auf ihre inhibitorische Aktivität für die Enzyme Cathepsin G, Leukozytenelastase, Organ-(Harn)-kallikrein, Plasmakallikrein und der Trypsine sind in Tabelle 4 zusammengestellt. Die Bestimmung der Enzymaktivitäten sowie die Hemmung der Enzyme wurde nach an sich bekannten Verfahren durchgeführt - vgl. Literaturangaben in Tabelle 4 -.

## Tabelle 4 — Bedingungen für die Testung diverser humaner Serinproteinasen

| Enzym | [Eo] (M) | Testpuffer* | Substrat | [So] (M) | Vorinku-bation (min) |
|---|---|---|---|---|---|
| Cathepsin G (human) | $2x10^{-7}$ | 0,2 M Tris; 50 mM $MgCl_2$; 0,05 % Tween 80, pH 7,2 | MeOSuc-Ala-Ala-Pro-Met-pNA[1] | $1x10^{-3}$ | 60 |
| Leukozytenelastase | $5x10^{-9}$ | 0,2 M Tris; | MeOSuc-Ala-Ala-Pro-Val-pNA[1] | $3x10^{-4}$ | 180 (human) |
| Organkallikrein (human) (Harn) | $2,5x10^{-8}$ | 0,2 M Tris; 0,05 % Tween 80, pH 8,2 | D-Val-Leu-Arg-pNAx2HCl[2] | $1,5x10^{-4}$ | 30 |
| Plasmakallikrein (human) | $3x10^{-9}$ | " | D-Pro-Phe-Arg-pNAx2HCl[3] | $4x10^{-4}$ | 120 |
| Trypsin (Rind) | $1x10^{-9}$ | 0,2 M Tris; 0,01 M $CaCl_2$; 0,05 % Tween 80, pH 8,0 | Pyr-Gly-Arg-pNA[4] | $2x10^{-5}$ | 90 |
| Trypsin anionisch (human) | " | " | " | " | " |
| Trypsin cationisch (human) | " | " | " | " | " |

* alle Testpuffer enthielten 0,05 % $NaN_3$

[1] K. Nakajima, J.C. Powers, B.M. Ashe und M. Zimmerman, J. Biol. Chem. 254, 4027-4032 (1979)

[2] E.Amundsen, J.Pütter, P.Friberger, M.Knos, M.Larsbraten und G.Claeson, Adv. Exp. Med. Biol. 120A (1979)

[3] A.M. Venneröd, K. Laake, A.K. Solberg und S. Strömland, Throm. Res. 9, 457 (1976)

[4] G. Claeson et al., Haemostasis 7, 76 (1978)

EP 0 297 362 A2

## Beispiele

### Beispiel 1

### Val-15-Human-Aprotinin

a) Konstruktion des Strukturgens von Val-15-Human-Aprotinin (Vektor pIU16.4.C)

Zur Genkonstruktion der Val-15-Human-Aprotinin-Variante wurden 1 nMol der komplementären Einzelstrang-Fragmente des entsprechenden $\alpha_1$, $\beta_1$, $\gamma_1$ und $\delta_1$-Genblocks in jeweils 200 $\mu$l Polynucleotidkinase-Reaktionspuffer aufgenommen und in Anwesenheit von ATP mit Polynucleotidkinase phosphoryliert - T. Manitis u.a., Molecular Cloning, Cold Spring Harbor (1982) -. Die DNA wurde mit Ethanol präzipitiert und je 1 nMol in 200 $\mu$l TE-Puffer-10 mM TrisxHCl-Puffer, 0,1 mM EDTA pH 7,5 - resuspendiert. Die komplementären DNA-Oligonucleotide wurden vereinigt und zur Hybridisierung in 400 $\mu$l TE-Puffer 5 Minuten auf 95° erhitzt und langsam im Wasserbad auf 30°C abgekühlt. Zur Ligation der hybridisierten Genblöcke $\alpha_1/\beta_1$ und $\gamma_1/\delta_1$ wurden je 500 pMol der zu ligierenden $\alpha_1 \cdot \beta_1$ bzw. $\gamma_1/\delta_1$ - Blöcke mit den Restriktionsenzymen EcoR I und Sty I bzw. mit Sty I und BamH I nachgeschnitten. Die so erzeugten Gensegmente wurden nach der präparativen Elektrophorese in 2 %igen Agarose Gelen isoliert und miteinander ligiert. Die ligierte DNA wurde mit den Restriktionsenzymen EcoR I und BamH I nachgespalten und das 200 bp Val-15-Human-Aprotinin-Gen durch präparative Elektrophorese in einem 2 %igen Agarose Gel gereinigt und dann aus dem Gel isoliert.

Das synthetische Val-15-Human-Aprotinin-Gen wurde in den Plasmidvektor pUC 8 kloniert - J. Vieira und J. Messing, Gene 19, 259 (1982) -. Dazu wurden 10 $\mu$g pUC 8-DNA mit EcoR I und BamH I gespalten und die DNA mit alkalischer Phosphatase (Kalb) dephosphoryliert. Die deletierte pUC 8-DNA wurde durch präparative Gelelektrophorese gereinigt und isoliert. 0,4 $\mu$g der so erhaltenen pUC 8-DNA und 0,1 $\mu$g Val-15-Human-Aprotinin-DNA wurden in 50 $\mu$l Ligationspuffer über Nacht mit 2 Einheiten T4-Ligase inkubiert. Mit der so rekombinierten DNA wurde der E.coli-Stamm RRIΔM15 transformiert. Die Selektion der Transformanten erfolgte auf LB-Platten mit 100 $\mu$g Ampicillin/ml.

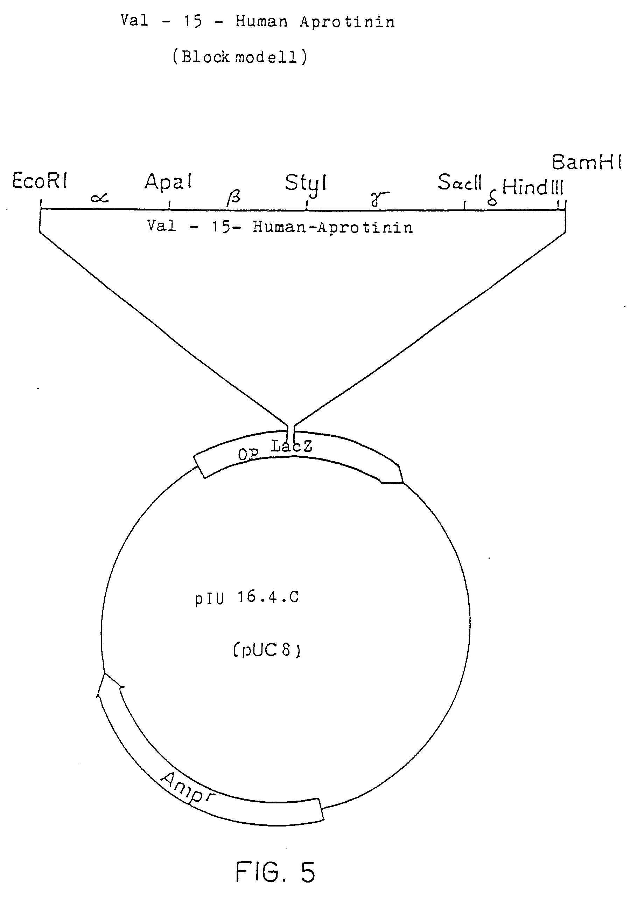

Von 100 Kolonien wurde die Plasmid-DNA nach der Methode von H.C. Birnboim und J. Doly - Nucl. Acid Res. 7, 1513 (1979) - isoliert und mit den Restriktionsenzymen EcoR I und BamH I analysiert. 3 der Plasmid-Klone hatten ein dem synthetischen Val-15-Human-Aprotinin in der Größe entsprechendes DNA-Fragment eingebaut. Die korrekte Sequenz der drei klonierten Val-15-Human-Aprotinin-Gene wurde bei der Sequenzierung bestätigt. Der Plasmid-Klon pIU 16.4.C (Abb. 5) wurde für die weiteren Konstruktionen verwendet.

b) Konstruktion des $\alpha$-Amylase-Sekretionsvektors pCH 237 und pCH 286

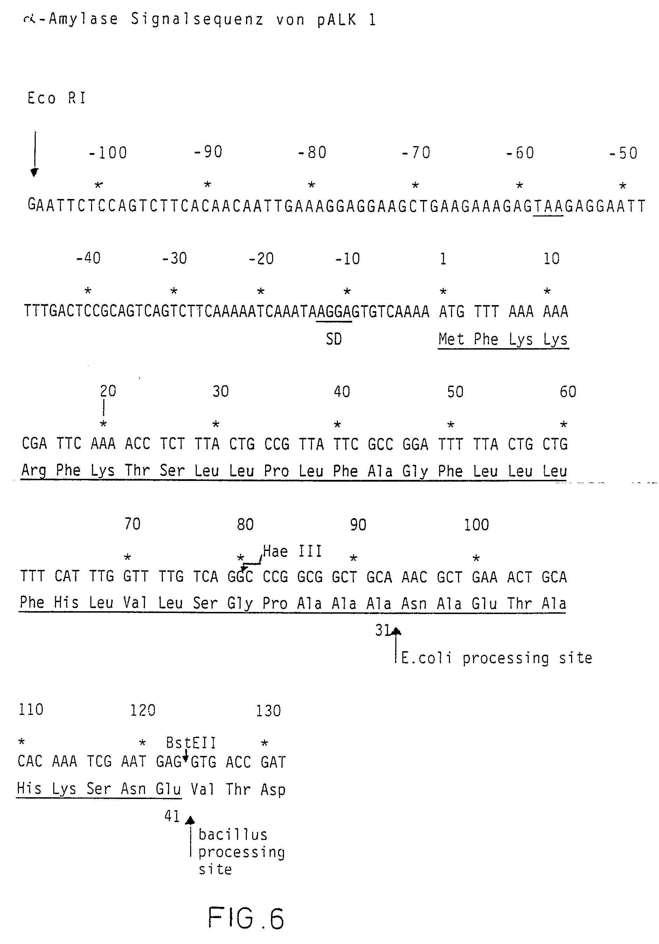

Ähnliche Vektorkonstruktionen sind in der Patentanmeldung GB-PS 8 700 204 vom 22.12.1986 beschrieben. Die $\alpha$-Amylase-Signalsequenz von B.subtilis wurde gewonnen aus DSM 704 (Deutsche Stammsammlung für Mikroorganismen, Göttingen) durch Klonierung eines partiellen Sau 3A Restrktionsansatzes der chromosomalen DNA in die BamH I site von pMK 3 - M.A. Sullivan et al., Gene 29, 21-26 (1984). Einer der Klone mit einem insertierten 3Kb DNA Fragment mit dem $\alpha$-Amylase-Gen wurde durch Deletionen im $\alpha$-Amylase-Strukturgen modifiziert und führte zu pALK 1 (persönl. Mitteilung von Dr. M.A. Courtney; Universität Rochester, Dept. of Microbiology, USA). DNA-Sequenzierungen eines 230 bp EcoR I -BstE II -Fragments von pALK 1 zeigten eine mögliche Ribosomen-Bindungsstelle (RBS) und ein Signalsequenz mit extensiver Homologie zu der $\alpha$-Amylase von B.subtilis 1A289 - vgl. DNA-Sequenz in Abb. 6 mit M. Yang u.a., Nucleic Acid Research 11, 237-249 (1983) -. Da die Prozessierung der $\alpha$-Amylase-Signalsequenz in E.coli am Aminosäurerest 31 erfolgt - W. Bruns, nicht veröffentlichte Resultate - wurde eine Nhe I-Restriktionsstelle am Alaninrest 31 eingeführt. Für diesen Zweck wurde aus pALK 1 - bezogen von Dr. M.A. Courtney, Rochester, USA - ein 180 bp EcoR I - Hae III - Fragment mit der RBS und dem größten Teil der α-Amylase-Signalsequenz (Fragment A in Abb. 6) isoliert. Fragment B, ein synthetischer Linker, der die Nhe I-site am Alanin 31 erzeugt, wurde zusammen mit Fragment A in pBR 322 zwischen EcoR I und Nhe I ligiert (pWB 226). pWB 226 wurde mit BamH I geschnitten, und nach einer Auffüllreaktion mit dNTP wurde ein Hind III - Linker (Biolabs 1002) in den Vektor ligiert und somit pWB 2024 erzeugt.

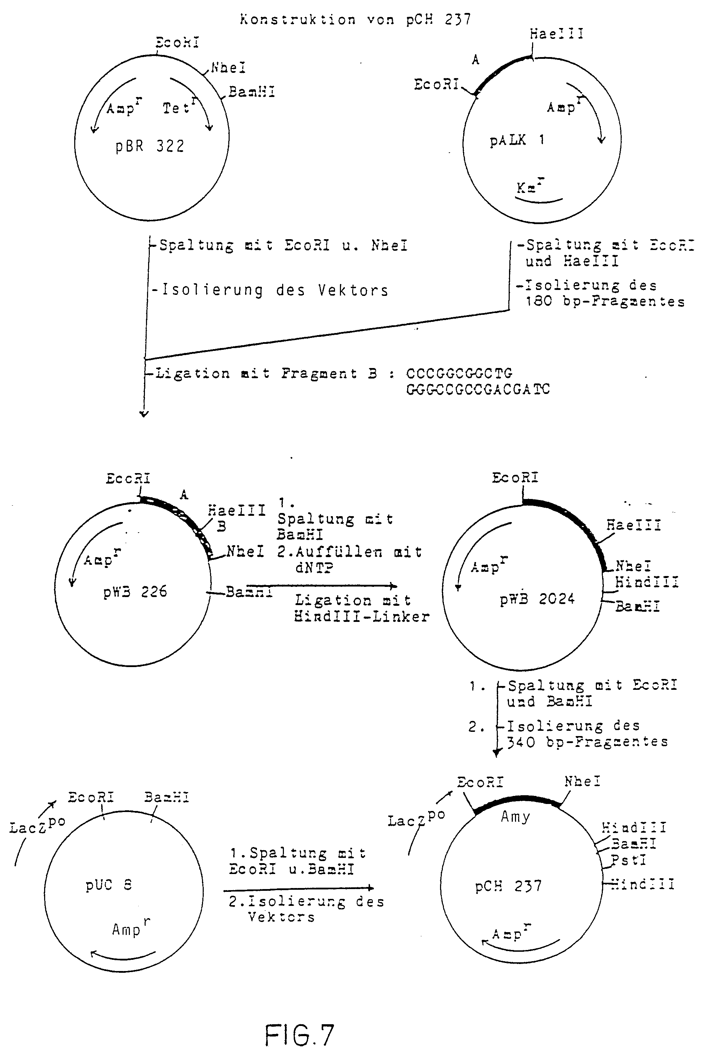

Der Sekretionsvektor pCH 237 wurde konstruiert, indem die A-Amylase-Signalsequenz unter die Kontrolle des lacZ Promoters in pUC 8 gebracht wurde (Abb. 7). In dieser Konstruktion wird lacZ' am TAA-Stopcodon (Pos. -58/-56 in der DNA-Sequenz der A-Amylase: Abb 6) gestoppt. Die Reinitiation der Proteinsynthese erfolgt dann an der möglichen RBS der α-Amylase ca. 50 Basen hinter dem Stopcodon von lacZ' an Pos. -10.

### c) Klonierung der Val-15-Human-Aprotinin codierenden Sequenz in den Sekretionsvektor pCH 237

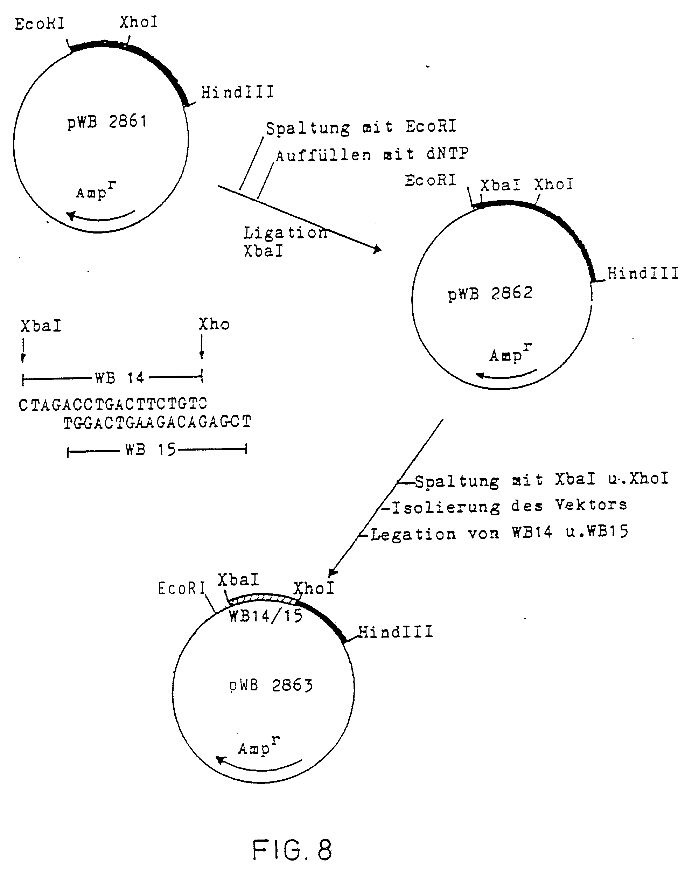

Für die Klonierung in den E.coli Sekretionsvektor pCH 237 wurden die Gensequenzen von Val-15-Human-Aprotinin (Abb. 4) als EcoR I - Hind III - Fragment in pUC 8 (zwischen EcoR I und Hind III) subkloniert (pWB 2861). pWB 2861 (Abb. 8) mit dem Gen für Val-15-Human-Aprotinin wurde mit EcoR I geschnitten und nach einer Auffüllreaktion mit dNTP wurde ein Xba-Linker (Biolabs 1010) in die Restriktionsstelle ligiert und dadurch pWB 2862 erzeugt. pWB 2862 wurde dann mit Xba I und Xho I geschnitten, der Vektor wurde isoliert und mit der synthetischen Linkersequenz WB 14 und 15 ligiert, wodurch eine Xba I-site am Codon für $Arg_1$ von Aprotinin (AGA) erzeugt wird (pWB 2863, Abb. 8).

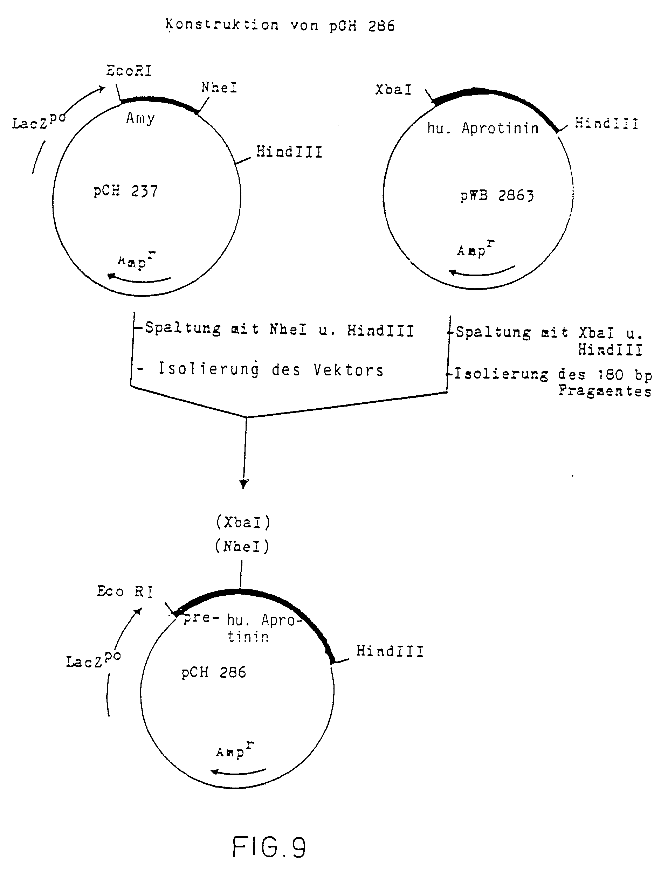

Für die Expression von Val-15-Human-Aprotinin wurde die Gensequenz als Xba I - Hind III - Fragment aus pWB 2863 isoliert und hinter die α-Amylase Signalsequenz in pCH 237 (geschnitten mit Nhe I und Hind III) integriert, was zur Konstruktion des Expressionsvektors pCH 286 führt (Abb. 9).

### d) Fermentation und Induktion von E.coli RR1ΔM 15 transformiert mit pCH 286

RR1ΔM 15 transformiert mit pCH 286 wurde als Übernachtkultur in 3 % Beef-Extrakt (Gibco), 1,5 % Hefe-Extrakt (Gibco) und 0,5 % $KH_2PO_4$ in destilliertem Wasser mit 4 % MES pH 6,0 und 50 μg/ml Ampicillin fermentiert (MES = Morpholinoethansulfonsäure). Für eine gute Belüftung wurden max. 100 ml Medium in 1.000 ml Erlenmeyerkolben verwendet und auf einer Kühner-Schüttelmachine (RC-6-U) bei 28° C und 190 UpM geschüttelt.

Für die Produktion von Val-15-Human-Aprotinin wurden Übernachtkulturen 1:100 in frischem Medium verdünnt und bei 28 oder 37° C für ca. 3 bis 5 Stunden fermentiert, bis zu einer $OD_{550nm}$ von etwa 1. Dann wurde durch Zugabe von 1 mM Isopropylthiogalactosid (Sigma) der lac Promoter induziert. Die Fermentation wurde über ca. 20 Stunden weitergeführt, wonach die Zellen durch Zentrifugation bei 8.000 UpM (10 Minuten bei 4° C in einer Kontron Centrikon H-401 mit Rotor A 6.14) geerntet wurden.

Die Zellen wurden suspendiert in 10 Vol. 0,05 M Trispuffer pH 7,5. Die Suspension wurde zum Zellaufschluß zweimal bei 18.000 PSI durch eine French Press passiert. Man trennte die Zelltrümmer durch Zentrifugieren - 30 Minuten, 8.000 g - ab und vereinigte das Zentrifugat mit dem Kulturfiltrat.

### e) Isolierung von Val-15-Human-Aprotinin

In 10 l einer Mischung von Kulturfiltrat und Zell-Lysat-Überstand - Stamm: RR1ΔM 15/pCH 286 - wurden bei Raumtemperatur langsam 350 ml 70 %iger Perchlorsäure eingerührt. Der dabei entstandene Niederschlag wurde nach 20 Minuten Stehen durch Zentrifugieren abgetrennt - 20 Minuten; 8.000 UpM; 4° C -. Dann wurde der Zentrifugationsüberstand mit 8 N Kaliumhydroxidlösung neutralisiert. Nach dem Stehen der Mischung über Nacht bei 4° C wurde das ausgefallene Kaliumperchlorat durch Filtration abgetrennt.

Die Filtrate wurden chromatographiert über eine mit 0.02 M HEPES-Puffer pH 6,5 (HEPES = 4-(2-Hydroxyethyl)-1-piperazin-ethansulfonsäure) äquilibrierte Säule - 20 x 2,5 cm -, die mit S-Sepharose, Fast Flow, beschickt war. Nach dem Auftrag wurde die Säule mit 250 ml Äquilibrierpuffer eluiert und schließlich mit einem linearen Gradienten aus je 500 ml Äquilibrierpuffer und 0,3 M Natriumchlorid enthaltendem Äquilibrierpuffer. Die mit 0,13 M bis 0,17 M NaCl eluierten Fraktionen enthielten elastaseinhibitorische Aktivität und wurden gepoolt und im Vakuum auf ein Volumen von 20 bis 30 ml konzentriert.

Das Konzentrat wurde zur weiteren Reinigung mittels HPLC über eine HiPore™ Reversed Phase Colum RP-318 (250 x 4,6 mm) - Bio-Rad, München; Katalog-Nr. 125-0551 - chromatographiert. Dazu wurde die HPLC-Säule zunächst nach dem Auftragen des Konzentrates und Nachspülen mit Wasser mit 0,1 %iger Trifluoressigsäure (Elutionsmittel A) eluiert und nach 10 Minuten mit einem linearen Gradienten aus Elutionsmittel A und 0,1 %iger Trifluoressigsäure, die 50 % Acetonitril enthielt. (Elutionsmittel B) -Gradient: 0 bis 100 % Elutionsmittel B von 0 bis 2 Stunden; Fließgeschwindigkeit 1 ml/min; Detektion bei 215 nm -.

Die UV-aktiven Fraktionen mit elastaseinhibitorischer Aktivität wurden vereinigt und ein Teil des Acetonitrils im Vakuum abgedampft. Bei der Lyophilisation des so erhaltenen Konzentrates wurden 3,8 mg einer farblosen Substanz erhalten, die in der analytischen HPLC:Mono S-Säule fast einheitlich war. Die Aminosäurezusammensetzung der Substanz ist aus Tabelle 3 ersichtlich, ihr Hemmspektrum ist in Tabelle 1 wiedergegeben. Die Aminosäuresequenzanalyse ergab, daß die Substanz zu ca. 78 % aus korrekt prozessiertem Val-15-Human-Aprotinin bestand und ca. 22 % Ala-(-1)-Val-15-Human-Aprotinin enthielt.

Beispiel 2

Val-15-Leu-17-Ile-18-Human-Aprotinin

a) Konstruktion des für Val-15-Leu-17-Ile-18-Thr-52-Human-Aprotinin codierenden Vektors pIU 24.4C

Die Synthese des Strukturgens erfolgte aus den Blöcken $\alpha_1$, $\beta_2$, $\gamma_1$ und $\delta_1$, wie bei Beispiel 1b) beschrieben. Die für Val-15-Leu-17-Ile-18-Thr-52-Human-Aprotinin codierende Sequenz wurde, wie bei Beispiel 1b) beschrieben, in pUC 8 eingebaut.

b) Konstruktion des α-Amylase-Sekretionsvektors pCH 287

Die Konstruktion des α-Amylase-Sekretions-Vektors pCH 287 erfolgt analog zur derjenigen des α-Amylase-Sekretionsvektors pCH 286.

c) Klonierung der für Val-15-Leu-17-Ile-18-Human-Aprotinin codierenden Sequenz in dem Sekretionsvektor pCH 237

Die Konstruktion des Expressionvektors pCH 287 mit der für Val-15-Leu-17-Ile-18-Human-Aprotinin codierenden Sequenz wurde nach dem gleichen Schema durchgeführt, wie für pCH 286 beschrieben.

d) Fermentation und Induktion von E.coli RR1ΔM 15 transformiert mit pCH 287

Die Fermentation des mit pCH 287 transformierten E.coli RR1ΔM 15 wurde durchgeführt, wie bei Beispiel 1c) beschrieben. Die Kulturbrühe wurde, wie dort beschrieben, durch Zentrifugieren geklärt und die Zellen analog aufgeschlossen. Zell-Lysat und Kulturfiltrat wurden schließlich vereinigt.

e) Isolierung von Val-15-Leu-17-Ile-18-Human-Aprotinin

Die vereinigten Zentrifugationsüberstände aus Beispiel 2d) - ca. 10 l - wurden bei Raumtemperatur im Verlauf von 10 Minuten unter Rühren mit 350 ml 70 %iger Perchlorsäure versetzt. Nach 20 Minuten Stehen wurde die entstandene Fällung durch Zentrifugieren abgetrennt. Das Zentrifugat wurde neutralisiert mit 8 N Natriumhydroxidlösung.

Die neutralisierte Lösung wurde bei 4°C über eine Säule - 10 x 2,5 cm - filtriert, die mit auf Sepharose 4B immobilisierten Anti-Aprotinin Antikörpern beschickt war und zuvor mit 0,1 M Natriumphosphatpuffer pH 7,0, 1 M an Natriumchlorid äquilibriert worden war. Die Säule wurde nach dem Beladen mit 100 ml Äquilibrierpuffer eluiert und das Aprotininderivat mit 0,2 M Glycin-Salzsäure-Puffer pH 2,4 desorbiert. Die Eluate wurde sofort mit gesättigter Trislösung neutralisiert und im Vakuum auf ein Volumen von ca. 20 ml eingeengt - Badtemperatur ~30° C -.

Zur Entsalzung und Abtrennung von geringen Proteinmengen wurden mit dem Konzentrat, wie bei Beispiel 1e) beschrieben, eine RP-318 HPLC durchgeführt. Die Eluate mit inhibitorischer Aktivität wurden vereinigt und das Acetonitril teilweise im Vakuum abdestilliert. Beim Lyophilisieren verblieben 3,2 mg farblose Substanz. Laut analytischer HPLC war der Inhibitor nahezu einheitlich. Das Inhibitionsspektrum der Substanz ist in Tabelle 1 wiedergegeben, das Ergebnis der quantitativen Aminosäureanalyse in Tabelle 3. Bei der Bestimmung der Aminosäuresequenz eine weitere Substanz gefunden - Anteil ca. 15 % - die einen zusätzlichen N-terminalen Ala-Rest enthielt.

In der analytischen FPLC - Lösungsmittel A: 0.02 M Natriumacetatpuffer pH 5.2; Lösungsmittel B: 0.02 M Natriumacetatpuffer pH 5,2; 0,3 M an Natriumchlorid - über eine Mono S-Pharmacia-Säule eluierte der Inhibitor bei 0.06 bis 0,08 M Natriumchlorid als einheitlicher Peak.

Beispiel 3

Val-15-Leu-17-Ile-18-Thr-52-Human-Aprotinin

a) Konstruktion des für den Inhibitor codierenden Strukturgens (Vektor pIU 26.4.C)

Die DNA-Blöcke $\alpha_1$, $\beta_2$, $\gamma$· und $\delta_2$ aus Tabelle 2 wurden, wie in Beispiel 1b) beschrieben, ligiert und ein den Vektor pUC 8 eingebaut. Der rekombinante Vektor erhielt die Bezeichnung pIU 26.4.C.

b) Fusion von lacZ-Gen und Val-15-Leu-17-Ile-18-Thr-52-Human-Aprotinin-Gen = Expressionsvektor pRK 151.1.8

Für die Expression der obigen Varianten von Human-Aprotinin als Fusionprotein mit der $\beta$-Galaktosidase von E.coli (lacZ-Gen) wurde der Plasmidvektor pUR 278 gewählt, in dem Genfusion mit dem für den C-Terminus codierenden Ende des lacZ-Gens erfolgt - U. Rüther und B. Müller-Hill, EMBO J. 2 1791 (1983) -. Die Expression des lacZ-Fusionsproteins erfolgt unter Kontrolle des lacZ-Promoters.

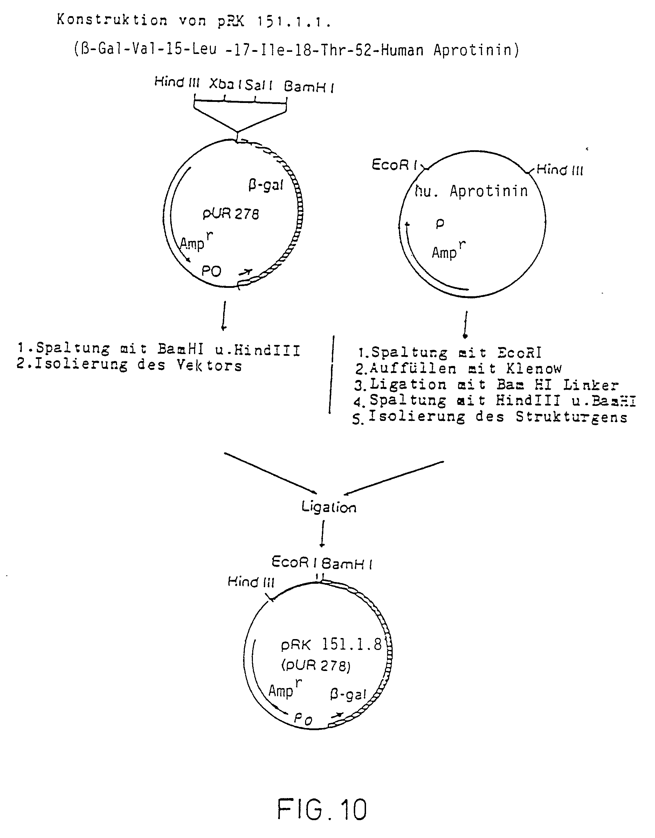

Die Insertion des Gens der Human-Aprotinin-Variante erfolgte zwischen den singulären BamH I und Hind III-Schnittstellen des lacZ-Gens von pUR 278. Dazu wurden 5 µg pUR 278-DNA mit BamH I und Hind III geschnitten und das große Vektorfragment isoliert (Abb. 10).

10 µg DNA des Vektors pIU 26.4.C wurden mit EcoR I linearisiert. Die überstehenden DNA-Enden wurden enzymatisch mit dem Klenow-Fragment der DNA-Polymerase in Anwesenheit von dATP und dTTP aufgefüllt. Die aufgefüllten DNA-Enden wurden mit einem 10fach molaren Überschuß an BamH I-Linkern (Pharmacia, Decamer) ligiert und das modifizierte Gen mit Hind III und mit BamH I aus dem Klonierungsvektor herausgespalten und nach der Reinigung durch Gelelektrophorese in einem 2 %igem Agarose-Gel isoliert.

Es wurden 0,2 µg Vektor-DNA und 0,1 µg der modifizierten pIU 26.4.C-DNA in 20 µg Ligationspuffer über Nacht mit 2 Einheiten T4-Ligase ligiert.

Die Transformation des E.coli-Stammes RR1ΔM 15 und die Selektion der rekombinierten Expressionsvektoren erfolgte nach Standardmethoden. In dem so erhaltenen Expressionsvektor pRK 151.1.8 wurde die Leserasterfusion des Inhibitorgens mit dem lacZ-Gen durch DNA-Sequenzierung bestätigt. Der E.coli-Stamm RR1ΔM 15/pRK 151.1.8 wurde für die Fermentation ausgewählt.

c) Fermentation

Die Fermentation des mit dem Plasmid pRK 151.1.8 transformierten E.coli-Stammes RR1ΔM 15 wurde bei 37° C durchgeführt, analog zu der im Beispiel 1d) beschriebenen Weise in LB-Ampicilin-Nährlösung und unter Schütteln - 280 UpM -.

22

d) Isolierung von Val-15-Leu-17-Ile-18-Thr-52-Human-Aprotinin

Aus 10 l Kulturbrühe - Fermentation des frisch mit IPTG induzierten Stammes RR1ΔM 15/pRK 151.1.8 -wurden die Zellen durch Zentrifugieren - 15 Minuten; 8.000 UpM - isoliert. Das Pellet - 23 g Naßgewicht - wurde in 50 ml 0,05 M Trispuffer, 0,1 M Natriumchlorid, 0,01 M Magnesiumchlorid und 0,01 M Mercaptoethanol pH 7,0 suspendiert. Die Zellen wurden in einer French Press aufgeschlossen - 18.000 PSI, zweimalige Passage - und das Lysat bei 20.000 UpM, 30 Minuten abgeschleudert. Das Pellet - ~18 g Naßgewicht - wurde resuspendiert in 100 ml 0,05 M Trispuffer pH 7,7, 2 M Guanidinium-hydrochlorid, 0,1 M Natriumchlorid, 0,01 M Mercaptoethanol und die Suspension durch 20 Minuten Zentrifugieren bei 20.000 g geklärt.

Das Pellet - 15 g Naßgewicht - wurde mit 50 ml 0,05 M Trispuffer pH 7,7, 6 M Guanidinium-hydrochlorid, 0,1 M Natriumchlorid extrahiert, der 500 mg Dithiothreitol enthielt. Die Suspension wurde dazu unter Rühren 1 Stunde auf 50° C erwärmt.

Man klärte die Suspension durch Zentrifugieren - 10 Minuten bei 10.000 UpM -. Das Zentrifugat wurde 24 Stunden gegen 10 l Wasser dialysiert, das 0,01 M Mercaptoethanol enthielt. Das dabei ausgefallene Fusionsprotein wurde durch Zentrifugieren - 20 Minuten; 20.000 UpM - isoliert.

Bromcyanspaltung

Das Pellet wurde unter Stickstoffatmosphäre in 30 ml Ameisensäure gelöst. Zu der Lösung fügte man 12,5 ml Wasser und anschließend 4 g Bromcyan. Man hielt die Reaktionsmischung unter Stickstoff und Lichtausschluß mit magnetischem Rühren 18 Stunden bei Raumtemperatur. Dann wurde die Reaktionslösung mit 100 ml Wasser verdünnt. Die Lösung wurde im Vakuum eingeengt, der Rückstand in Wasser gelöst, erneut im Vakuum eingeengt und schließlich in Wasser gelöst und lyophilisiert. Es verblieben 1,8 g farblose Substanz.

Renaturierung

600 mg der obigen Substanz wurden suspendiert in 300 ml 0,02 M Natriumacetatpuffer pH 5,2, der 6 M an Harnstoff und 0,01 M an Mercaptoethanol war (Startpuffer). Man setzt der Suspension 36 g Harnstoff und 3 ml Mercapoethanol und erwärmte sie 1 Stunde auf 50° C. Die Suspension wurde durch Zentrifugieren geklärt und das Zentrifugat über Nacht gegen 25 Vol. des Startpuffers dialysiert. In das Retentat wurden 15 ml mit dem Startpuffer äquilibrierte S-Sepharose, Fast Flow eingerührt und die Suspension nach 30 Minuten in eine mit 5 ml äquilibrierter S-Sepharose, Fast Flow, gefüllte Säule - 1,5 x 12 cm - eingeschlämmt. Die Säule wurde mit Startpuffer gewaschen, bis die Eluate optisch leer waren. Dann wurde eluiert mit einem linearen Gradienten aus 180 ml Startpuffer und 180 ml 0,02 M Natriumacetatpuffer pH 5,2, der 0,002 M an Mercaptoethanol war. Man eluierte anschließend mit 0,02 M Natriumacetatpuffer pH 5,2 (Waschpuffer), bis die Eluate proteinfrei waren und schließlich mit 0,5 M Natriumchlorid/l enthaltendem obigen Waschpuffer.

Reinigung

Die elastaseinhibitorische Aktivität enthaltenden Eluate wurden vereinigt und ihr Volumen durch Einengen im Vakuum auf 15 ml konzentriert. Das Konzentrat wurde, wie bei Beispiel 1d) beschrieben, mittels HPLC über eine HiPore™ RP-Säule chromatographiert unter Verwendung eines Gradienten aus 0,1 %iger Trifluoressigsäure und 50 % Acetonitril enthaltender 0,1 %iger Trifluoressigsäure. Die nach dem Lyophilisieren der inhibitorisch aktiven Eluate erhaltene Substanz - ca. 10 mg - wurde durch FPLC über eine Mono S-Säule chromatographiert unter Anwendung eines linearen Gradienten aus 0,02 M Natriumacetatpuffer pH 5,2 und 0,02 M Natriumacetatpuffer pH 5,2, der 0,3 M an Natriumchlorid war. Die mit 0,05 M - 0,07 M Natriumchlorid eluierte Substanz wurde durch HPLC über eine RP-318-Säule entsalzt, wie bereits mehrfach beschrieben, und war in der analytischen HPLC praktisch einheitlich. Die Ausbeute betrug 0,5 mg. Das Inhibitionsspektrum des Inhibitors ist in Tabelle 1 verglichen mit dem von Human-Aprotinin; die quantitative Aminosäurezusammensetzung ist aus Tabelle 3 ersichtlich. Durch Sequenzierung wurde die erwartete Sequenz bestätigt.

Beispiel 4

Leu-15-Leu-17-Ile-18-Thr-52-Human-Aprotinin

a) Konstruktion des für den Inhibitor codierenden Strukturgens (Vektor pIU 2.6.C)

Die DNA-Blöcke $\alpha_1$, $\beta_3$, $\gamma_1$ und $\delta_2$ nach Tabelle 2 wurden ligiert, wie im Beispiel 1b) beschrieben. Nach elektrophoretischer Reinigung wurde pIU 2.6.C erhalten.

b) Konstruktion des Expressionsvektors für das Fusionsprotein von Leu-15-Leu-17-Ile-18-Thr-52-Human-Aprotinin mit der MS2-Replikase (Vektor pRK 152.1.1)

Für die Expression von Leu-15-Leu-17-Ile-18-Thr-52-Human-Aprotinin als Fusionsprotein mit einer Teil sequenz der MS2-Replikase wurde der Vektor pEx 31.b. (Strebel et. al, J. of Virology (1986) 57,983-991) ausgewählt. Die Expression des MS2-Fusionsproteins wird in diesem Vektor durch den $P_L$-Promoter des Phagen Lambda kontrolliert.

5 µg pIU 2.6.C-DNA wurde mit EcoR I und BamH I geschnitten und das Gen der Human-Aprotinin-Variante wie üblich isoliert. Es wurden 0,1 µg der dabei erhaltenen DNA mit 0,4 mg DNA des pEx 31.b-Vektors ligiert. Die pEx 31.b-DNA war zuvor mit EcoR I und BamH I erhalten und mit alkalischer Phosphatase (Kalb) dephosphoryliert worden.

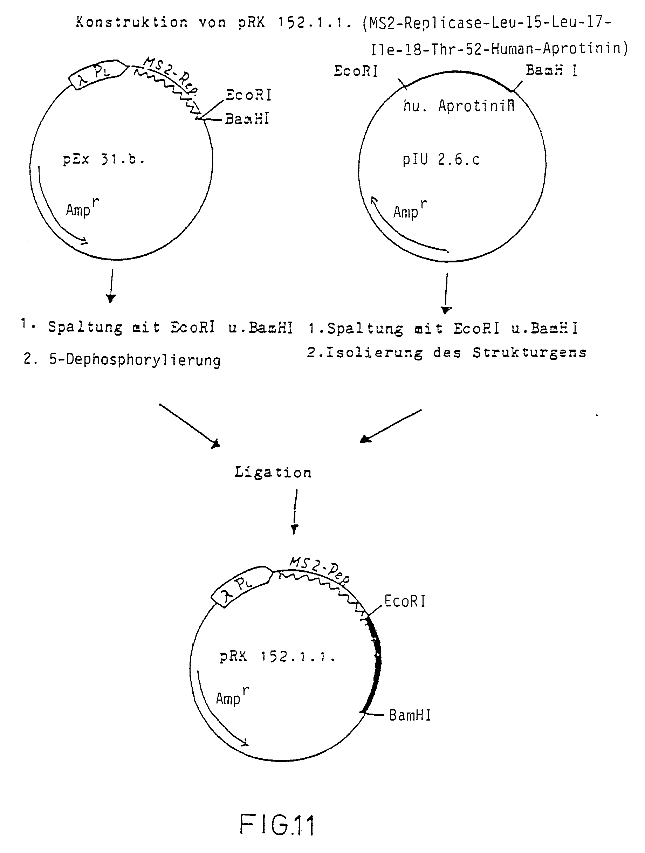

Die Transformation des E.coli-Stammes 537 mit der rekombinanten DNA sowie die anschließenden Klon-Analysen erfolgten nach Standardverfahren. Die korrekte "in frame"-Fusion der klonierte Human-Aprotinin-Genvariante mit dem fusionierten MS2-Gen wurde durch Sequenzanalyse des Plasmidklons pRK 152.1.1 (Abb. 11) bestätigt.

Fermentation

Die Fermentation des mit dem Plasmid pRK 152.1.1 transformierten Stammes E.coli 537 wurde durchgeführt in einer auf pH 7 eingestellten Nährlösung aus 30 g/l Hefeextrakt, 30 g/l Fleischextrakt und 1 g Dikaliumhydrogenphosphat/l entionisiertem Wasser. Nach dem Sterilisieren der Nährlösung wurden jeweils 100 ml Portionen in 1 l Schüttelkolben mit einer im gleichen Medium angezogenen 8 Stunden-Vorkultur angeimpft. Man hielt 4 Stunden bei 28°C unter Schütteln in einem Rotationsschüttler bei 280 UpM. Dann wurde 3 Stunden auf 42°C erhitzt, wobei die Ausbeute an Fusionsprotein auf 15 % des Proteingehalts der Zelle - SDS/PAGE -anstieg.

d) Isolierung von Leu-1-5-Leu-17-Ile-18-Thr-52-Human-Aprotinin

Die Zellen wurde durch Zentrifugieren - 15 Minuten; 5.000 UpM - isoliert. Man suspendierte das Pellet in 10 Volumina 0,1 M Tris-Salzsäurepuffer pH 7,5, 0,01 M Ethylendiamintetraessigsäure, 0,005 M Mercaptoethanol und 0,005 M Benzamidinhydrochlorid und inkubierte die Suspension mit gutem Rühren nach Zugabe von Lysozym (Fluka AG, Buchs, Schweiz) - 0,2 mg/ml - 30 Minuten bei 30°C. Die Reaktionsmischung wurde dann auf 4°C gekühlt und mit der French Press (Amico, USA) bei 18.000 PSI aufgeschlossen. Das Homogenat wurde 30 Minuten bei 10.000 UpM - Beckmann JA-10 - zentrifugiert und das Zentrifugat verworfen. Das Pellet wurde zweimal in obigen Puffer resuspendiert, der 2 M an Harnstoff war, und die jeweils erhaltene Suspension erneut durch Zentrifugieren geklärt.

## Isolierung des Fusionsproteins

Das so erhaltene Sediment wurde anschließend unter magnetischem Rühren mit 0,05 M Trispuffer pH 8,5, 8 M an Guanidinium-hydrochlorid und 0,01 M an Mercaptoethanol ausgelaugt und die unlöslichen Anteile durch Zentrifugieren abgetrennt - 30 Minuten, 18.000 UpM -. Das Zentrifugat wurde in Portionen von je 10 ml über eine Sephacryl S-300-Säule (5 x 90 cm) filtriert, die mit 0,05 Tris-salzsäurepuffer pH 8,5, 6 M an Harnstoff und 0,01 M an Mercaptoethanol äquilibriert war und mit dem Äquilibrierpuffer eluiert wurde. Die das Fusionsprotein enthaltenden Fraktionen - Identifizierung mittels SDS-PAGE unter reduzierenden Bedingungen - wurden gepoolt und erschöpfend gegen Wasser dialysiert. Dabei fiel das Fusionsprotein nahezu quantitativ aus und wurde durch Zentrifugieren - 30 Minuten; 10.000 UpM - isoliert.

## Bromcyanspaltung

500 mg Fusionsprotein wurden in 21 ml Ameisensäure gelöst und nach Zugabe von 9 ml Wasser und 500 mg Bromcyan 18 Stunden unter Stickstoffatmosphäre und Lichtausschluß bei Raumtemperatur gehalten. Dann wurde die Lösung mit 300 ml Wasser versetzt und Lösungsmittel sowie überschüssiges Bromcyan im Vakuum abdestilliert. Man löste den Eindampfrückstand in 50 ml 0,05 M Natriumacetatpuffer, 6 M Harnstoff und 0,01 M Mercaptoethanol, pH 5,2, unter Zusatz von 6 g Harnstoff. Diese Lösung wurde über Nacht gegen 20 Vol. des Lösepuffers dialysiert.

## Renaturierung

Das Retentat wurde auf eine mit CM-Sepharose, Fast Flow, beschickte und mit dem Lösepuffer äquilibrierte Säule - 2,5 x 10 cm - aufgetragen und die Säule zunächst mit Lösepuffer eluiert und - schließlich mit einem linearen Gradienten aus 300 ml Lösepuffer und 0,05 M Natriumacetatpuffer, 0,002 M Mercaptoethanol, pH 5,2, und schließlich mit 0,05 M Natriumacetatpuffer, pH 5,2. Der renaturierte Inhibitor wurde schließlich mit 0,05 M Acetatpuffer pH 5,2, 0,5 M an NaCl von der Säule desorbiert.

## Reinigung

Die Fraktionen mit elastaseinhibitorischer Aktivität wurden vereinigt und erschöpfend gegen 0,020 M Natriumacetatpuffer, pH 5,2, dialysiert. Man trug das Retentat auf eine mit dem Dialysepuffer äquilibrierte Mono-S-FPLC-Säule auf und entwickelte die Säule mit einem linearen Gradienten aus jeweils 100 ml Äquilibrierpuffer und 0,3 M Natriumchlorid enthaltendem Äquilibrierpuffer. Die den Inhibitor enthaltenden Fraktionen wurden durch präparative HPLC über eine RP-318 Säule in der bereits beschriebenen Weise entsalzt. Der Inhibitor wurde aus den Eluaten durch Lyophilisation nach dem teilweisen Abdampfen des Acetonitrils erhalten. Ausbeute 9,5 mg farblose Substanz. Das Inhibitionsspektrum der Präparation ist aus Tabelle 1 ersichtlich, die quantitative Aminosäurezusammensetzung aus Tabelle 3. Die erwartete Sequenz wurde bei der Sequenzierung über 22 Schritte bestätigt.

## Beispiel 5

## Arg-15-Arg-17-Ile-18-Glu-52-Human-Aprotinin

## a) Konstruktion des für den Inhibitor codierenden Strukturgens (Vektor = pIU 4.6.C)

Durch Ligieren der synthetischen Genblöcke $\alpha_1$, $\beta_4$, $\gamma_2$ and $\delta_3$ von Tabelle 2 nach dem im Beispiel 1b) beschriebenen Verfahren wurde das pUC 8-Derivat mit pIU 4.6.C erhalten.

25

b) Konstruktion des Expressionsvektors für das Fusionsprotein von Arg-15-Arg-17-Ile-18-Glu-52-Human-Aprotinin mit der MS2-Replikase (Vektor pRK 153.1.1)

Die Fusion der Gene für MS2-Replikase und Arg-15-Arg-17-Ile-18-Glu-52-Human-Aprotinin wurde analog zu der im Beispiel 4b) beschriebenen Fusion durchgeführt und ergab den Vektor pRK 153.1.1.

c) Fermentation des mit pRK 153.1.1 transformierten E.coli-Stammes 537

Die Fermentation wurde durchgeführt wie für E.coli 537/pRK 152.1.1 im Beispiel 4c) beschrieben.

d) Isolierung von Arg-15-Arg-17-Ile-18-Arg-39-Glu-52-Human-Aprotinin

Analog zu Beispiel 4d) wurden die nach der Fermentation des mit dem Plasmid pRK 153.1.1 transformierten E.coli-Stammes 537 erhaltenen Zellen isoliert und aufgeschlossen.

Die gewaschenen Einschlußkörper wurde in der ebenfalls im Beispiel 4d) beschriebenen Weise einer Bromcyanspaltung unterworfen. Der Inhibitor wurde an CM-Sepharose bei pH 5.2 regeneriert. Die Reinigung erfolgte durch Chromatographie an Mono-S (FPLC) mit einem linearen Gradienten aus 0,02 M HEPES pH 6.0 und 0.02 M HEPES, 0,3 M Natriumchlorid pH 6,0. In den Fraktionen wurde der Inhibitor anhand seiner antitryptischen Aktivität nachgewiesen. Die Elution erfolgte bei 0,15 bis 0,18 M Natriumchlorid. In Tabelle 1 ist das Inhibitionsspektrum des Inhibitors ersichtlich, die quantitative Aminosäureanalyse aus Tabelle 3. Die erwartete Sequenz wurde bei der Sequenzierung über 24 Schritte bestätigt.

**Ansprüche**

1. Human-Aprotinin, dessen Lys-Rest in Position 15 gegen einen anderen protogenen Aminosäurerest ausgetauscht ist.

2. Human-Aprotinin gemäß Anspruch 1, bei dem ein oder mehrere zuzätzliche Aminosäurereste gegen andere protogene Aminosäurereste ausgetauscht sind.

3. Human-Aprotinin gemäß Anspruch 2, mit einem Austausch in einer oder mehreren der Positionen 14, 16, 17, 18, 19, 34, 38, 39 und 52.

4. Human-Aprotinin, mit einem Austausch des Lys-Restes in Position 15 gegen einen Aminosäurerest aus der Gruppe Val, Ile, Le, Ala. Met. Asn. Gln, Phe, Tyr, Trp und Arg.

5. Human-Aprotinin gemäß Ansprüchen 1 bis 4, worin für den Fall eines Austausches in den jeweiligen Positionen jeweils die folgenden Aminosäurereste auftreten können:
Position 16:
Gly, Ser oder Thr
Position 17:
Leu, Val, Ile, Ser, Thr, Asn, Gln, Phe, Tyr oder Arg
Position 18:
Ala. Val. Leu. Ile. Ser. Thr. Asn. Gln. Asp, Glu oder Phe
Position 19:
Val, Ile, Leu, Ala, Thr, Asn oder Gln
Position 34:
Ile, Leu, Ala, Thr, Met, Asn, Gln, Asp, Glu, Phe oder Arg
Position 39:
Val, Ile. Leu. Met. Asn. Gln, Asp, Glu, Phe, Ser. Thr oder Arg
Position 52:
Val, Ile. Leu. Gln. Glu. Thr, Asp, Phe, Lys oder Arg.
und in den Positionen 14 und 38: Gly, Ala, Val, Leu, Pro, Ser, Thr, Asn, Gln, Glu, Lys, Arg oder Phe, wobei die Aminosäurereste in diesen beiden Positionen gleich oder verschieden sein können.

6. Nucleinsäure, die für Human-Aprotinin gemäß den Ansprüchen 1 bis 5 codiert.

7. Expressionssystem, enthaltend eine DNA nach Anspruch 6.

8. Verfahren zur Herstellung von Human-Aprotinin gemäß den Ansprüchen 1 bis 5, dadurch gekennzeichnet, daß man eine Nucleinsäure, die für das Human-Aprotinin codiert, in einen Wirtsorganismus einbringt, der das Human-Aprotinin exprimiert und anschließend das Expressionsprodukt isoliert.

9. Arzneimittel, enthaltend Human-Aprotinin gemäß den Ansprüchen 1 bis 5.

10. Verwendung von Human-Aprotinin gemäß den Ansprüchen 1 bis 5 zur Herstellung von Arzneimitteln.

FIG.1

FIG. 2

Erfindungsgemäße DNA-Blöcke für die Synthese der Gene

```
          CTGCGTTGCTAAAATGATCCGTTACTTCTACAATGC
β₁  ------+---------+---------+---------+---
          CCGGGACGCAACGATTTTACTAGGCAATGAAGATGTTACGGTTC


          CTGCGTTGCTCTGATCATCCGTTACTTCTACAATGC
β₂  ------+---------+---------+---------+-------
          CCGGGACGCAACGAGACTAGTAGGCAATGAAGATGTTACGGTTC


          CTGCCTGGCTCTGATCATCCGTTACTTCTACAATGC
β₃  ------+---------+---------+---------+-------
          CCGGGACGGACCGAGACTAGTAGGCAATGAAGATGTTACGGTTC


          CTGCCGTGCTCGTATCATCCGTTACTTCTACAATGC
β₄  ------+---------+---------+---------+-------
          CCGGGACGGCACGAGCATAGTAGGCAATGAAGATGTTACGGTTC


     CAAGGCAGGCTTCTGTGAAACCTTCGTATACGGCGGTTGCAAAGCTAAGAGCAACAACTTCCGTTCCGC
γ₁  ------+---------+---------+---------+---------+---------+---------+--
     CGTCCGAAGACACTTTGGAAGCATATGCCGCCAACGTTTCGATTCTCGTTGTTGAAGGCAAGG


     CAAGGCAGGCTTCTGTGAAACCTTCGTATACGGCGGTTGCCGTGCTAAGAGCAACAACTTCCGTTCCGC
γ₂  ------+---------+---------+---------+---------+---------+---------+--
     CGTCCGAAGACACTTTGGAAGCATATGCCGCCAACGGCACGATTCTCGTTGTTGAAGGCAAGG


     GGAAGACTGCATGCGTACTTGCGGTGGTGCTTAGTAAAGCTTG
δ₁  --------+---------+---------+---------+---------
     CGCCTTCTGACGTACGCATGAACGCCACCACGAATCATTTCGAACCTAG


     GGAAGACTGCACCCGTACTTGCGGTGGTGCTTAGTAAAGCTTG
δ₂  --------+---------+---------+---------+---------
     CGCCTTCTGACGTGGGCATGAACGCCACCACGAATCATTTCGAACCTAG
```

FIG. 3

Sequenz des Met-(-1)-Val-15-Human-Aprotinin-Gens mit
den Schnittstellen für die Restriktionsenzyme

```
    E
    c                          X                    A
    o                          h                    p
    R                          o                    a
    1                          1                    1
    GAATTCATGCGTCCGGACTTCTGCCTCGAGCCGCCGTACACTGGGCCCTGCGTTGCTAAA
1   ---------+---------+---------+---------+---------+---------+   60
    CTTAAGTACGCAGGCCTGAAGACGGAGCTCGGCGGCATGTGACCCGGGACGCAACGATTT
                               S                    A
                               t                    c
                               y                    c
                               1                    1
    ATGATCCGTTACTTCTACAATGCCAAGGCAGGCTTCTGTGAAACCTTCGTATACGGCGGT
61  ---------+---------+---------+---------+---------+---------+  120
    TACTAGGCAATGAAGATGTTACGGTTCCGTCCGAAGACACTTTGGAAGCATATGCCGCCA
                               S                    S
                               a                    p
                               c                    h
                               2                    1
    TGCAAAGCTAAGAGCAACAACTTCCGTTCCGCGGAAGACTGCATGCGTACTTGCGGTGGT
121 ---------+---------+---------+---------+---------+---------+  180
    ACGTTTCGATTCTCGTTGTTGAAGGCAAGGCGCCTTCTGACGTACGCATGAACGCCACCA
                H        B
                i        a
                n        m
                d        H
                3        1
    GCTTAGTAAAGCTTGGATCC
161 ---------+---------+  200
    CGAATCATTTCGAACCTAGG
```

FIG. 4

Val – 15 – Human Aprotinin

(Block modell)

FIG. 5

α-Amylase Signalsequenz von pALK 1

Eco RI

|
↓    -100      -90      -80      -70      -60      -50
      *        *        *        *        *        *

GAATTCTCCAGTCTTCACAACAATTGAAAGGAGGAAGCTGAAGAAAGAGTAAGAGGAATT

   -40      -30      -20      -10      1      10
    *      *      *      *      *      *

TTTGACTCCGCAGTCAGTCTTCAAAAATCAAATAAGGAGTGTCAAAA ATG TTT AAA AAA

                             SD           Met Phe Lys Lys

   20      30      40      50      60
    *      *      *      *      *

CGA TTC AAA ACC TCT TTA CTG CCG TTA TTC GCC GGA TTT TTA CTG CTG
Arg Phe Lys Thr Ser Leu Leu Pro Leu Phe Ala Gly Phe Leu Leu Leu

   70      80      90     100
    *      * Hae III  *      *

TTT CAT TTG GTT TTG TCA GGC CCG GCG GCT GCA AAC GCT GAA ACT GCA
Phe His Leu Val Leu Ser Gly Pro Ala Ala Ala Asn Ala Glu Thr Ala

                          31▲
                           E.coli processing site

110      120      130
*          * BstEII   *
CAC AAA TCG AAT GAG GTG ACC GAT
His Lys Ser Asn Glu Val Thr Asp

         41▲
          bacillus
          processing
          site

FIG.6

FIG.7

FIG. 8

Konstruktion von pCH 286

FIG.9

Konstruktion von pRK 151.1.1.

(ß-Gal-Val-15-Leu -17-Ile-18-Thr-52-Human Aprotinin)

Hind III  Xba I Sal I  BamH I

β-gal

pUR 278

Amp$^r$

PO

EcoR I    Hind III

hu. Aprotinin

p

Amp$^r$

1.Spaltung mit BamHI u.HindIII
2.Isolierung des Vektors

1.Spaltung mit EcoRI
2.Auffüllen mit Klenow
3.Ligation mit Bam HI Linker
4.Spaltung mit HindIII u.BamHI
5.Isolierung des Strukturgens

Ligation

EcoR I BamH I

Hind III

pRK 151.1.8
(pUR 278)

Amp$^r$    β-gal

Po

FIG. 10

Konstruktion von pRK 152.1.1. (MS2-Replicase-Leu-15-Leu-17-
Ile-18-Thr-52-Human-Aprotinin)

1. Spaltung mit EcoRI u.BamHI
2. 5-Dephosphorylierung

1.Spaltung mit EcoRI u.BamHI
2.Isolierung des Strukturgens

Ligation

FIG.11